# EUROPEAN PATENT APPLICATION

(11) **EP 4 847 533 A1**
(43) Date of publication of application: **02.12.2026**
(21) Application number: 25745384.5
(22) Date of filing: 24.01.2025
(51) Int. Cl.: C07K 16/30, C07K 14/725, C12N 5/12, C12N 5/0783, G01N 33/574, A61K 40/11, A61K 40/31, A61K 40/42, A61P 35/00

(54) **NOVEL ANTIBODY SPECIFICALLY BINDING TO GPC3 AND USE THEREOF**

(30) Priority: 24.01.2024 KR 20240010948
(71) Applicant: National Cancer Center, Goyang-si, Gyeonggi-do 10408 (KR)
(72) Inventor: HAN, Chungyong, Incheon 22003 (KR); SEO, Ho Kyung, Goyang-si Gyeonggi-do 10403 (KR); LEE, Sang-Jin, Goyang-si Gyeonggi-do 10579 (KR); CHOI, Beom Kyu, Paju-si Gyeonggi-do 10893 (KR); KIM, Hye Ran, Goyang-si Gyeonggi-do 10416 (KR); JUNG, Eui Hyun, Seoul 07362 (KR); KIM, Doyeon, Yongin-si Gyeonggi-do 16908 (KR)
(74) Representative: Isarpatent
(86) International application number: PCT/KR2025/001458
(87) International publication number: WO 2025/159562

(57) **Abstract**

The present disclosure relates to: an antibody specifically binding to GPC3; a CAR-T using same; a humanized antibody of the antibody; and a use of a CAR-T using the humanized antibody. A novel antibody specifically binding to GPC3 protein and a fragment or humanized antibody thereof having immunological activity according to the present disclosure specifically binds to GPC3, involved in signaling (YAP and Wnt/b-catenin signaling) playing an important role in cancer formation/progression, and exhibits an anticancer immune response, and thus can be used as an antibody therapeutic agent for cancer treatment and as a cell therapeutic agent using cancer cell-killing mechanisms via immune cell activation, and can also be used for cancer diagnosis.

## Description

### [Technical Field]

The present disclosure relates to a novel antibody specifically binding to GPC3, a chimeric antigen receptor T cell (CAR-T) using the same, and uses thereof.

### [Background Art]

Glypican 3 (GPC3) is known as a type of oncofetal antigen belonging to a glypican group of glycosyl-phosphatidylinositol-immobilized heparin sulfate proteoglycans, and cell membrane-bound glypican-3 is known to consist of two subunits linked by one or more disulfide bonds.

The GPC3 expression is not confirmed in adult tissues other than the placenta, but is confirmed in various cancer tissues such as hepatocellular carcinoma (HCC), hepatoblastoma, melanoma, ovarian clear cell carcinoma, Wilm's tumor, and lung squamous cell carcinoma. As such, the GPC3 is classified as an oncofetal antigen because of a protein expressed in embryonic tissue, just like proteins such as α-fetoprotein (AFP) and carcinoembryonic antigen (CEA). In other words, the GPC3 is not expressed in normal tissue cells, but exhibits the characteristic of being specifically expressed in cancer cells, and thus can be utilized as a target molecule for cancer treatment, as well as a tumor marker and a diagnostic marker.

Furthermore, currently developed medicines for treatment are largely divided into low molecular medicines and high molecular medicines, and the high molecular medicines with specificity are in the spotlight as therapeutic agents compared to the low molecular medicines with relatively large side effects without specificity.

Among them, protein therapeutic agents have been widely used in clinical trials by rapidly replacing non-specific low molecular compound therapeutic agents due to very high specificity and low side effects and toxicity for disease targets. Particularly, among protein therapeutic agents which have been currently used in clinical trials, antibody therapeutic agents make up the majority. Therapeutic antibodies are considered as one of the most effective cancer treatment methods by exhibiting very high target specificity, and having not only low biotoxicity and few side effects, but also an excellent blood half-life of about 3 weeks compared to conventional low molecular drugs. In fact, large pharmaceutical companies and research institutes around the world are accelerating the research and development of therapeutic antibodies that specifically bind to cancer cells, including cancer-causing factors, to effectively remove the cancer cells. Companies for developing therapeutic antibody drugs mainly consist of pharmaceutical companies, such as Roche, Amgen, Johnson & Johnson, Abbott, and BMS. Particularly, Roche includes representative products of Herceptin, Avastin, Rituxan, and the like for anticancer treatment, and not only produces large profits, achieving sales of approximately $19.5 billion in the global market in 2012 with these three therapeutic antibodies, but also leads the global antibody drug market. Johnson & Johnson, which developed Remicade, is also growing rapidly in the global antibody market due to increased sales, and pharmaceutical companies such as Abbott and BMS are also known to have many therapeutic antibodies in the final stages of development. As a result, in the global pharmaceutical market, where low molecular drugs had been dominated, the low molecular drugs have been rapidly replaced with biopharmaceuticals including therapeutic antibodies that are specific to disease targets and have low side effects.

Meanwhile, unlike conventional immunotherapy, there has been developed an immune cell-based cancer immunotherapy that specifically binds to cancer cells and eliminates the cancer cells by immune cells in the body. The cancer immunotherapy is a cancer therapeutic agent that includes T cells (CAR T cells) that express a chimeric antigen receptor (CAR) capable of directly recognizing tumor-associated antigens in cells, or T cells that express a specific antibody sequence against an immune checkpoint receptor of cancer cells in the cells, and is produced to enable immune cells to specifically attack cancer cells. Human T cell therapy depends on enriched or modified human T cells to target and kill cancer cells in patients. To increase the ability of T cells to target and kill specific cancer cells, a method for engineering T cells has been developed to express constructs that direct T cells to specific target cancer cells. A chimeric antigen receptor (CAR) and an engineered T cell receptor (TCR) containing a binding domain capable of interacting with specific tumor antigens enable T cells to target and kill cancer cells expressing specific tumor antigens. The human T cell therapy is a cell therapy in which a sequence of an antibody binding to a CAR-T cell tumor antigen (e.g., CD19) and the like binds to a domain necessary for T cell signaling, such as CD3/4-1BB/CD28 to be inserted into T cells. There are various methods for inserting these CAR genes into T cells, but most methods use a lentivirus delivery system. The structure of currently developed chimeric antigen receptors is divided into a single chain variable fragment (scFv) portion that recognizes antigens, a transmembrane domain, and a signaling domain that transmits signals into the cells. Currently, pharmaceutical companies, such as Novartis, Gilead, Celgene, and Abclon, along with clinicians, have developed CAR-T cells using scFv that specifically bind to a CD19 antigen of blood cancer cells as a treatment for blood cancer, but the development of CAR-T cells that show a more definite anticancer effect has been required for the complete conquest of cancer.

### [Disclosure]

### [Technical Problem]

An object of the present disclosure is to provide a GPC3-specific antibody or a fragment having immunological activity thereof.

Another object of the present disclosure is to provide a humanized antibody specifically binding to GPC3 or an antigen binding fragment thereof.

Yet another object of the present disclosure is to provide a hybridoma cell line.

Yet another object of the present disclosure is to provide a nucleic acid molecule.

Yet another object of the present disclosure is to provide a vector.

Yet another object of the present disclosure is to provide a transformed host cell.

Yet another object of the present disclosure is to provide a composition for detecting cancer antigens.

Yet another object of the present disclosure is to provide a kit for detecting cancer antigens.

Yet another object of the present disclosure is to provide an antibody-drug conjugate (ADC).

Yet another object of the present disclosure is to provide a pharmaceutical composition for preventing or treating cancer.

Yet another object of the present disclosure is to provide a composition for diagnosing cancer.

Yet another object of the present disclosure is to provide a kit for diagnosing cancer.

Yet another object of the present disclosure is to provide a method for providing information for the diagnosis of cancer.

Yet another object of the present disclosure is to provide a GPC3-specific chimeric antigen receptor (CAR).

Yet another object of the present disclosure is to provide a GPC3-specific humanized antibody-derived chimeric antigen receptor (CAR).

Yet another object of the present disclosure is to provide a polynucleotide.

Yet another object of the present disclosure is to provide a vector.

Yet another object of the present disclosure is to provide a method for producing a GPC3-specific CAR or a GPC3-specific humanized antibody-derived CAR.

Yet another object of the present disclosure is to provide a chimeric antigen receptor expressing cell.

Yet another object of the present disclosure is to provide a pharmaceutical composition for preventing or treating cancer.

Yet another object of the present disclosure is to provide a cell therapeutic agent for preventing or treating cancer.

Yet another object of the present disclosure is to provide an anticancer adjuvant.

Yet another object of the present disclosure is to provide a composition for diagnosing cancer.

Yet another object of the present disclosure is to provide a method for providing information for the diagnosis of cancer.

Yet another object of the present disclosure is to provide a method for detecting cancer antigens.

Yet another object of the present disclosure is to provide a method for preventing or treating cancer.

Yet another object of the present disclosure is to provide a method for enhancing an anticancer treatment effect in a subject.

### [Technical Solution]

In order to achieve the object, the present disclosure provides an isolated antibody specifically binding to a Glypican-3 (GPC3) protein or a fragment having immunological activity thereof.

In addition, the present disclosure provides a humanized antibody specifically binding to GPC3 or an antigen binding fragment thereof, including: a human VH domain including any one or more CDRHs selected from the group consisting of complementarity determining regions Heavy chain (CDRH)1 including an amino acid sequence represented by SEQ ID NO: 1, CDRH2 including an amino acid sequence represented by SEQ ID NO: 2, and CDRH3 including an amino acid sequence represented by SEQ ID NO: 3; and a human VL domain including any one or more CDRLs selected from the group consisting of CDRL1 including an amino acid sequence represented by SEQ ID NO: 4, CDRL2 including an amino acid sequence represented by SEQ ID NO: 5, and CDRL3 including an amino acid sequence represented by SEQ ID NO: 6.

In addition, the present disclosure provides a hybridoma cell line that produces an isolated antibody or a fragment having immunological activity thereof according to the present disclosure, or a humanized antibody or an antigen binding fragment thereof according to the present disclosure.

In addition, the present disclosure provides an isolated nucleic molecule that encodes an isolated antibody or a fragment having immunological activity thereof according to the present disclosure, or a humanized antibody or an antigen binding fragment thereof according to the present disclosure.

In addition, the present disclosure provides a vector including an isolated nucleic molecule that encodes an isolated antibody or a fragment having immunological activity thereof according to the present disclosure, or a humanized antibody or an antigen binding fragment thereof according to the present disclosure.

In addition, the present disclosure provides a host cell transformed with a vector including an isolated nucleic molecule that encodes an isolated antibody or a fragment having immunological activity thereof according to the present disclosure, or a humanized antibody or an antigen binding fragment thereof according to the present disclosure.

In addition, the present disclosure provides a composition for detecting cancer antigens including an isolated antibody or a fragment having immunological activity thereof according to the present disclosure, or a humanized antibody or an antigen binding fragment thereof according to the present disclosure.

In addition, the present disclosure provides a kit for detecting cancer antigens including an isolated antibody or a fragment having immunological activity thereof according to the present disclosure, or a humanized antibody or an antigen binding fragment thereof according to the present disclosure.

In addition, the present disclosure provides an antibody-drug conjugate (ADC) including an isolated antibody or a fragment having immunological activity thereof according to the present disclosure, or a humanized antibody or an antigen binding fragment thereof according to the present disclosure; and a drug.

In addition, the present disclosure provides a pharmaceutical composition for preventing or treating cancer including an antibody-drug conjugate according to the present disclosure.

In addition, the present disclosure provides a composition for diagnosing cancer including an isolated antibody or a fragment having immunological activity thereof according to the present disclosure, a humanized antibody or an antigen binding fragment thereof according to the present disclosure, or an antibody-drug conjugate according to the present disclosure.

In addition, the present disclosure provides a kit for diagnosing cancer including an isolated antibody or a fragment having immunological activity thereof according to the present disclosure, a humanized antibody or an antigen binding fragment thereof according to the present disclosure, or an antibody-drug conjugate according to the present disclosure.

Furthermore, the present disclosure provides a method for providing information for the diagnosis of cancer including: treating a sample with an isolated antibody or a fragment having immunological activity thereof according to the present disclosure, a humanized antibody or an antigen binding fragment thereof according to the present disclosure, or an antibody-drug conjugate according to the present disclosure, which is labeled with a fluorescent substance; and measuring the expression of the isolated antibody or the fragment having immunological activity thereof, the humanized antibody or the antigen binding fragment thereof, or the antibody-drug conjugate.

To solve the object, the present disclosure provides a GPC3-specific chimeric antigen receptor (CAR) including: as an antigen binding domain, a single chain variable fragment (scFv) including a heavy chain variable region including complementarity determining regions heavy chain 1 (CDRH1) including an amino acid sequence represented by SEQ ID NO: 1, CDRH2 including an amino acid sequence represented by SEQ ID NO: 2, and CDRH3 including an amino acid sequence represented by SEQ ID NO: 3; and a light chain variable region including CDRL1 including an amino acid sequence represented by SEQ ID NO: 4, CDRL2 including an amino acid sequence represented by SEQ ID NO: 5, and CDRL3 including an amino acid sequence represented by SEQ ID NO: 6.

Further, the present disclosure provides a GPC3-specific humanized antibody-derived chimeric antigen receptor (CAR) including: as an antigen binding domain, a single chain variable fragment (scFv) including any one heavy chain variable region selected from the group consisting of a heavy chain variable region including an amino acid sequence represented by SEQ ID NO: 11; a heavy chain variable region including an amino acid sequence represented by SEQ ID NO: 12; a heavy chain variable region including an amino acid sequence represented by SEQ ID NO: 13; and a heavy chain variable region including an amino acid sequence represented by SEQ ID NO: 14; and any one light chain variable region selected from the group consisting of a light chain variable region including an amino acid sequence represented by SEQ ID NO: 15; a light chain variable region including an amino acid sequence represented by SEQ ID NO: 16; a light chain variable region including an amino acid sequence represented by SEQ ID NO: 17; and a light chain variable region including an amino acid sequence represented by SEQ ID NO: 18.

In addition, the present disclosure provides a polynucleotide including a nucleic acid sequence encoding a GPC3-specific CAR according to the present disclosure or a GPC3-specific humanized antibody-derived CAR according to the present disclosure.

In addition, the present disclosure provides a vector including a polynucleotide including a nucleic acid sequence encoding a GPC3-specific CAR according to the present disclosure or a GPC3-specific humanized antibody-derived CAR according to the present disclosure.

In addition, the present disclosure provides a method for producing a GPC3-specific CAR or a GPC3-specific humanized antibody-derived CAR, including infecting a host cell with a vector including a polynucleotide including a nucleic acid sequence encoding a GPC3-specific CAR according to the present disclosure or a GPC3-specific humanized antibody-derived CAR according to the present disclosure.

In addition, the present disclosure provides a chimeric antigen receptor expressing cell transformed with a GPC3-specific CAR according to the present disclosure.

In addition, the present disclosure provides a chimeric antigen receptor expressing cell transformed with a GPC3-specific humanized antibody-derived CAR according to the present disclosure.

In addition, the present disclosure provides a pharmaceutical composition for preventing or treating cancer including a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

In addition, the present disclosure provides a cell therapeutic agent for preventing or treating cancer including a chimeric antigen receptor expressing cell transformed with a GPC3-specific CAR according to the present disclosure or a chimeric antigen receptor expressing cell transformed with a GPC3-specific humanized antibody-derived CAR according to the present disclosure.

In addition, the present disclosure provides an anticancer adjuvant including a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

In addition, the present disclosure provides a composition for diagnosing cancer including a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

In addition, the present disclosure provides a method for providing information for the diagnosis of cancer including: treating a sample with a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure, which is labeled with a fluorescent substance; and measuring the expression of the GPC3-specific CAR, the GPC3-specific humanized antibody-derived CAR, the chimeric antigen receptor expressing cell transformed with the GPC3-specific CAR, or the chimeric antigen receptor expressing cell transformed with the GPC3-specific humanized antibody-derived CAR.

In addition, the present disclosure provides a method for detecting cancer antigens including: administering, to a subject, an isolated antibody or a fragment having immunological activity thereof according to the present disclosure, a humanized antibody or an antigen binding fragment thereof according to the present disclosure, a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

In addition, the present disclosure provides a method for preventing or treating cancer, including administering, to a subject, an antibody-drug conjugate according to the present disclosure, a GPC3-specific CAR according to the present disclosure, a GPC3-specific humanized antibody-derived CAR according to the present disclosure, a chimeric antigen receptor expressing cell transformed with a GPC3-specific CAR according to the present disclosure, a chimeric antigen receptor expressing cell transformed with a GPC3-specific humanized antibody-derived CAR according to the present disclosure, a cell therapeutic agent including a chimeric antigen receptor expressing cell transformed with a GPC3-specific CAR according to the present disclosure, or a cell therapeutic agent including a chimeric antigen receptor expressing cell transformed with a GPC3-specific humanized antibody-derived CAR according to the present disclosure.

In addition, the present disclosure provides a method for enhancing an anticancer treatment effect in a subject including: administering, to a subject, a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

### [Advantageous Effects]

The present disclosure relates to: an antibody specifically binding to GPC3; a CAR-T using the same; a humanized antibody of the antibody; and a use of a CAR-T using the humanized antibody. A novel antibody specifically binding to GPC3 protein, a fragment having immunological activity thereof or a humanized antibody thereof according to the present disclosure specifically binds to GPC3, involved in signaling (Yap and Wnt/b-catenin signaling) playing an important role in cancer formation/progression, and exhibits an anticancer immune response, and thus can be used as an antibody therapeutic agent for cancer treatment and as a cell therapeutic agent using cancer cell-killing mechanisms via immune cell activation, and can also be used for cancer diagnosis.

### [Description of Drawings]

FIG. 1 is a diagram showing a process of preparing a GPC3 (Glypican-3)-specific antibody:
   Left: Immuno-induced GPC3 protein; and
   Right: Process for deriving GPC3-specific hybridoma antibody clones.
FIG. 2 is a diagram showing results of ELISA analysis for specificity to GPC3 using a supernatant of each hybridoma single-cell clone.
FIG. 3 is a diagram showing GPC3-specific binding of selected 10-1.4 antibody clones by flow cytometry:
   Unstained: unstained group;
   Anti-GPC3 Ab: Positive control group stained using anti-human GPC3:APC antibody;
   Isolated Ab clone + 2' Ab: Group primary stained with a culture supernatant of selected hybridoma antibody clone 10-1.4 and then secondary stained with an anti-mouse IgG:PE antibody; and
   2' Ab only: Group secondary stained with an anti-mouse IgG:PE antibody without primary staining.

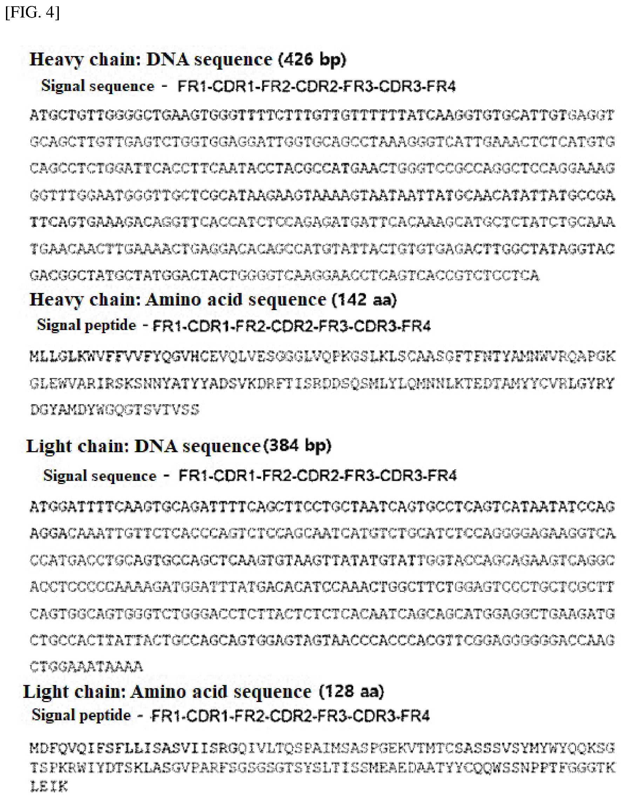

FIG. 4 is a diagram showing a sequencing result of a VH region (Signal sequence-FR1-CDR1-FR2-CDR2-FR3-CDR3-FR4) and a VL region (Signal sequence-FR1-CDR1-FR2-CDR2-FR3-CDR3-FR4) of a GPC3-specific hybridoma antibody clone 10-1.4 of the present disclosure.

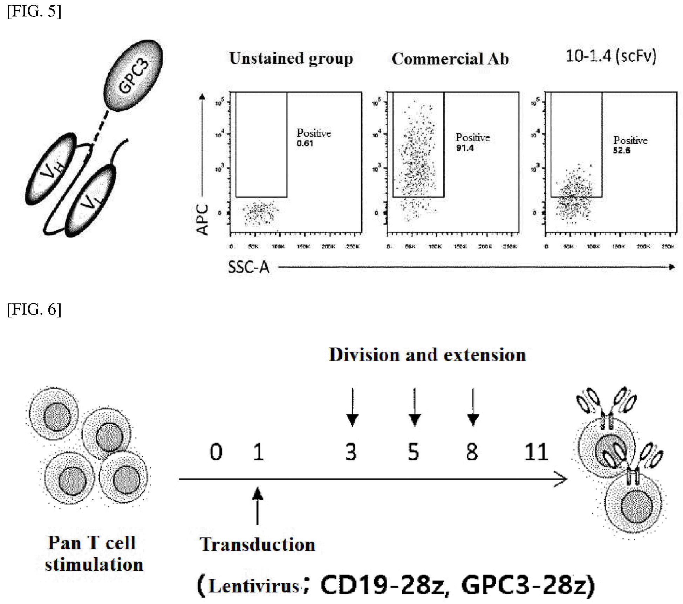

FIG. 5 is a diagram showing GPC3-specific binding of an scFv format antibody of a hybridoma antibody clone 10-1.4 of the present disclosure by flow cytometry:
   Left: scFv format of hybridoma antibody clone 10-1.4;
   Right: Flow cytometry results;
   Unstained: unstained group;
   Commercial Ab: Positive control group stained using anti-human GPC3:APC antibody; and 10-1.4 (scFv): Group primary stained with a FLAG tag-scFv format protein of selected hybridoma clone 10-1.4 and then secondary stained with an anti-FLAG tag:APC antibody.
FIG. 6 is a diagram showing a process of preparing a GPC3 CAR-T or CD19 (negative control) CAR-T of the present disclosure.

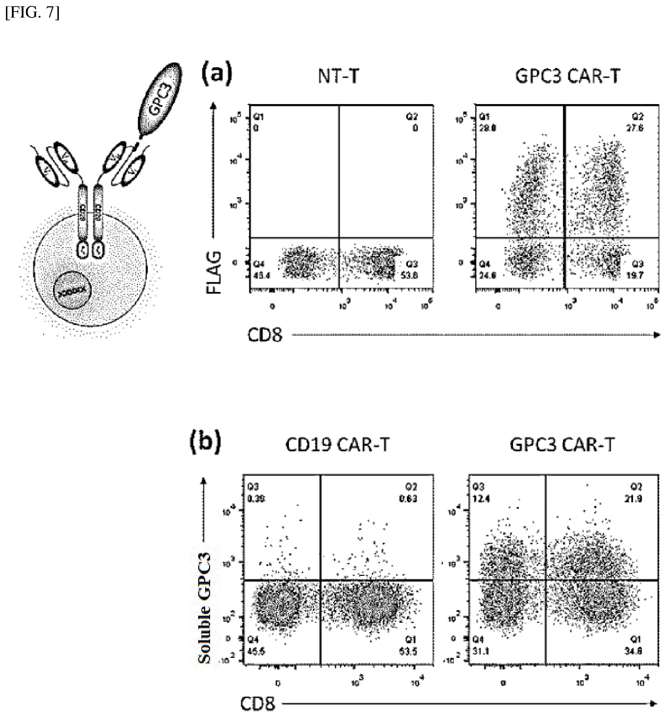

FIG. 7 is a diagram showing antigen specificity (a) and binding to a GPC3 antigen (b) of GPC3 CAR-T by flow cytometry:
   NT-T: Non-transduced T cell group (group not introduced with GPC3 CAR);
   GPC3 CAR-T: T cell group introduced with GPC3 CAR;
   CD19 CAR-T: CAR-T with scFv of an FMC63 antibody clone recognizing CD19 (negative control);
   GPC3 CAR-T: T cell group introduced with GPC3 CAR; and
   Left diagram: Schematic diagram of a T cell introduced with a CAR including GPC3 scFv of the present disclosure as an antigen binding domain.

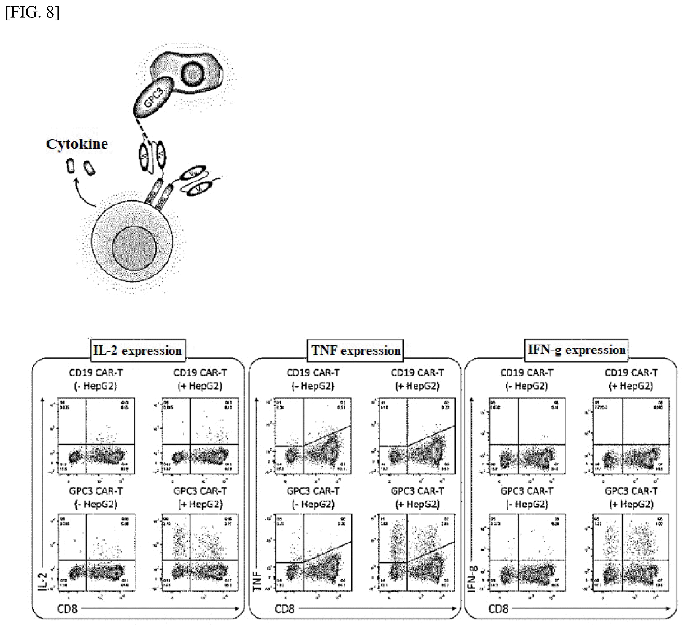

FIG. 8 is a diagram showing a GPC3-specific anticancer immune response of GPC3 CAR-T confirmed through release of cytokines:
   CD19 CAR-T (- HepG2): CD19 CAR-T cultured alone;
   CD19 CAR-T (+ HepG2): CD19 CAR-T co-cultured with HepG2;
   GPC3 CAR-T (- HepG2): GPC3 CAR-T cultured alone;
   GPC3 CAR-T (+ HepG2): GPC3 CAR-T co-cultured with HepG2; and
   Upper diagram: Schematic diagram of release of cytokines after binding with GPC3 of GPC3 CAR-T of the present disclosure.

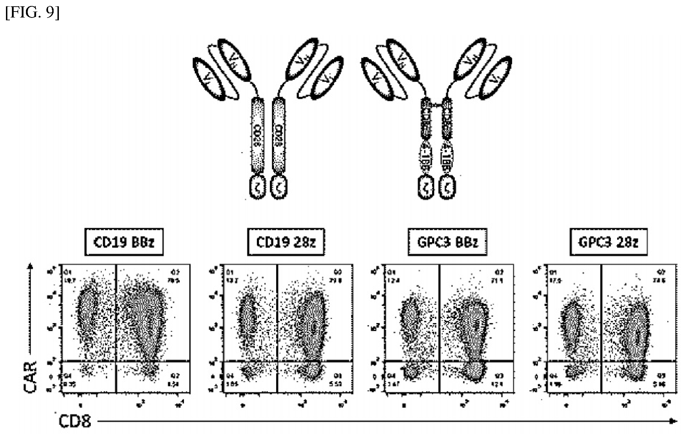

FIG. 9 is a diagram showing CAR-T cells including a GPC3 scFv of the present disclosure as an antigen binding domain and having CD28 or 4-1BB as a co-stimulatory domain, and cell surface expression thereof:
   CD19 BBz: CAR-T having scFv of an FMC63 antibody clone used as a control and a 4-1BB co-stimulatory domain;
   CD19 28z: CAR-T having scFv of an FMC63 antibody clone used as a control and a CD28 co-stimulatory domain;
   GPC3 BBz: CAR-T having an scFv sequence of clone 10-1.4 and a 4-1BB co-stimulatory domain; and
   GPC3 28z: CAR-T having an scFv sequence of clone 10-1.4 and a CD28 co-stimulatory domain.

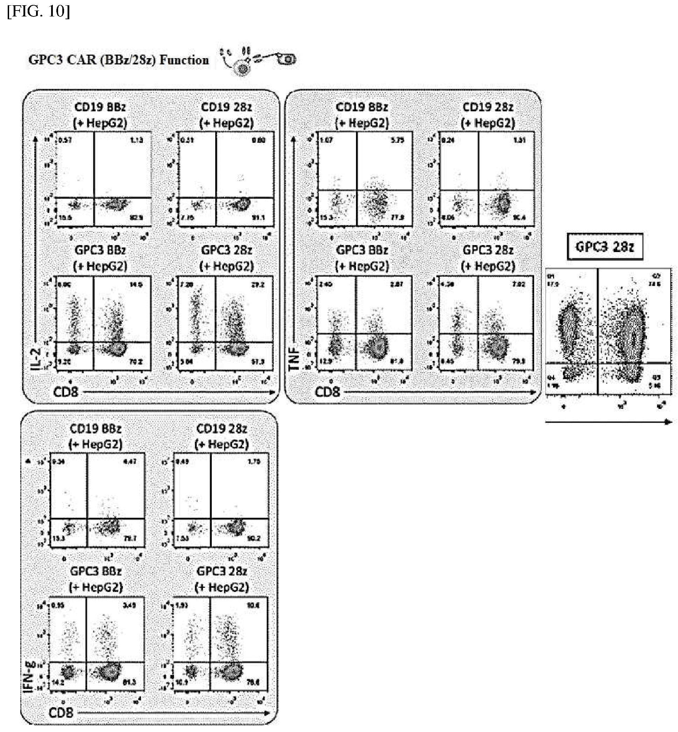

FIG. 10 is a diagram showing GPC3-specific anticancer immune responses of CAR-T cells including a GPC3 scFv of the present disclosure as an antigen binding domain and having CD28 or 4-1BB as a co-stimulatory domain:
   CD19 BBz: CAR-T having scFv of an FMC63 antibody clone used as a control and a 4-1BB co-stimulatory domain;
   CD19 28z: CAR-T having scFv of an FMC63 antibody clone used as a control and a CD28 co-stimulatory domain;
   GPC3 BBz: CAR-T having an scFv sequence of clone 10-1.4 and a 4-1BB co-stimulatory domain; and
   GPC3 28z: CAR-T having an scFv sequence of clone 10-1.4 and a CD28 co-stimulatory domain.

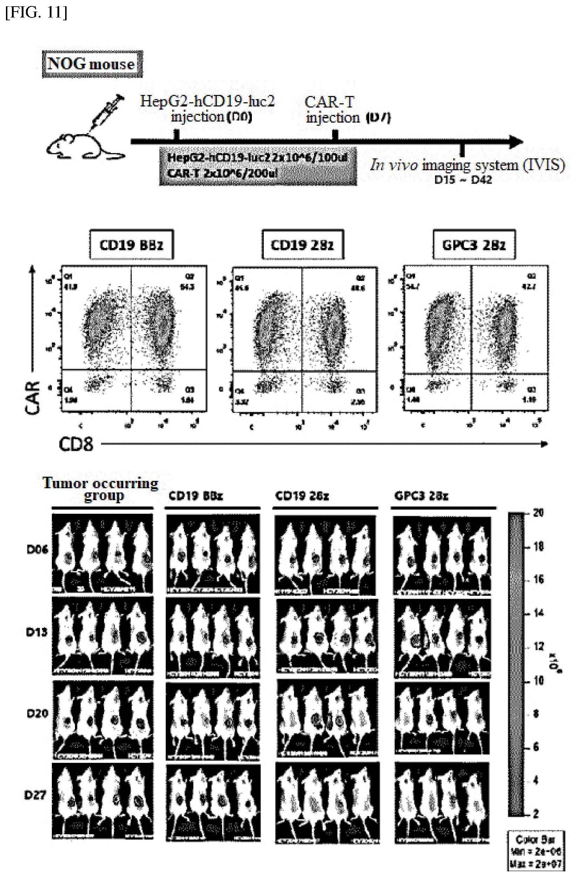

FIG. 11 is a diagram showing anticancer efficacy after administering, to a liver cancer-induced animal model, CAR-T cells including a GPC3 scFv of the present disclosure as an antigen binding domain and having CD28 or 4-1BB as a co-stimulatory domain:
   CD19 BBz: CAR-T having scFv of an FMC63 antibody clone used as a control and a 4-1BB co-stimulatory domain;
   CD19 28z: CAR-T having scFv of an FMC63 antibody clone used as a control and a CD28 co-stimulatory domain; and
   GPC3 28z: CAR-T having an scFv sequence of clone 10-1.4 and a CD28 co-stimulatory domain.

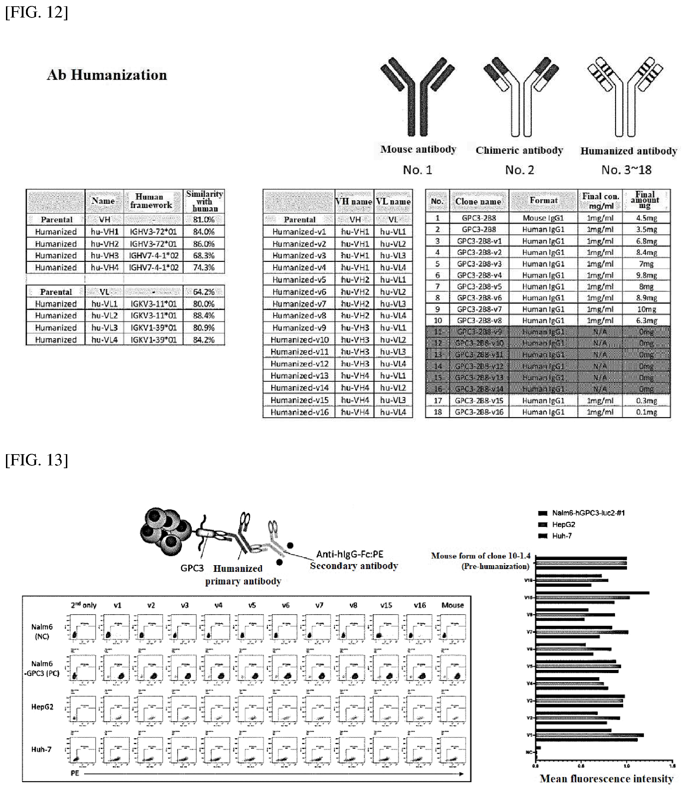

FIG. 12 is a schematic diagram showing preparation of a humanized antibody grafted with a CDR sequence of a GPC3-specific hybridoma antibody clone 10-1.4 of the present disclosure.
FIG. 13 is a diagram showing GPC3 antigen specificity of a humanized antibody grafted with a CDR sequence of a GPC3-specific hybridoma antibody clone 10-1.4 of the present disclosure.

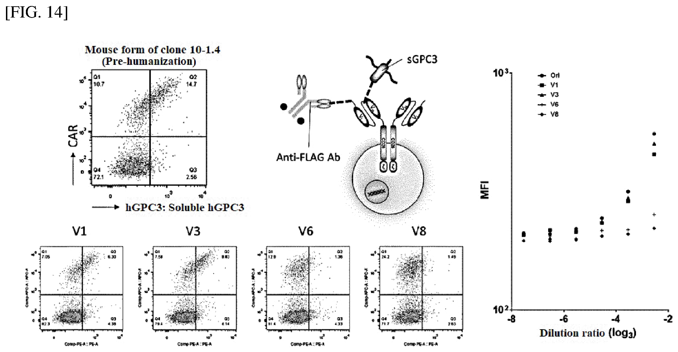

FIG. 14 is a schematic diagram showing a T cell introduced with a CAR including, as an antigen binding domain, scFv of a humanized antibody grafted with a CDR sequence of a GPC3-specific hybridoma antibody clone 10-1.4 of the present disclosure, and a diagram showing GPC3 antigen specificity thereof.

### [Best Mode]

Hereinafter, the present disclosure will be described in detail by exemplary embodiments of the present disclosure with reference to the accompanying drawings. However, the following exemplary embodiments are provided by illustration of the present disclosure. When it is determined that the specific description of known techniques or configuration well known to those skilled in the art unnecessarily obscures the gist of the present disclosure, the detailed description therefor may be omitted, and the present disclosure is not limited thereto. The present disclosure allows various modifications and applications within the description of claims to be described below and the scope of equivalents interpreted therefrom.

Further, terminologies used herein are terminologies used to properly represent preferred exemplary embodiments of the present disclosure, which may vary depending on the intent of users or operators, or custom in the art to which the present disclosure pertains. Therefore, these terminologies used herein will be defined based on the contents throughout the specification. Throughout this specification, unless explicitly described to the contrary, when a certain part "comprises" a certain component, it is meant to further comprise other components rather than excluding other components.

Unless otherwise defined, all the technical terms used herein are used with the same meaning as commonly understood by those skilled in the art in the field related to the present disclosure. In addition, although preferred methods and samples are described herein, those similar or equivalent thereto are incorporated in the scope of the present disclosure. The contents of all the publications described herein as references are incorporated in the present disclosure.

Throughout the specification, not only conventional one-letter or three-letter codes for naturally occurring amino acids are used, but also generally allowed three-letter codes for other amino acids, such as *α*-aminoisobutyric acid (Aib) and N-methylglycine (Sar), are also used. The amino acids mentioned herein as abbreviations are also described according to the IUPAC-IUB Nomenclature below.

Alanine: A; Arginine: R; Asparagine: N; Aspartic acid: D; Cysteine: C; Glutamic acid: E; Glutamine: Q; Glycine: G; Histidine: H; Isoleucine: I; Leucine: L; Lysine: K; Methionine: M; Phenylalanine: F; Proline: P; Serine: S; Threonine: T; Tryptophan: W; Tyrosine: Y; and Valine: V.

In an aspect, the present disclosure relates to an isolated antibody specifically binding to a Glypican-3 (GPC3) protein or a fragment having immunological activity thereof.

In an exemplary embodiment, the isolated antibody or the fragment having immunological activity thereof may include a VH domain including complementarity determining regions heavy chain 1 (CDRH1) including an amino acid sequence represented by SEQ ID NO: 1, CDRH2 including an amino acid sequence represented by SEQ ID NO: 2, and CDRH3 including an amino acid sequence represented by SEQ ID NO: 3.

In an exemplary embodiment, the isolated antibody or the fragment having immunological activity thereof may include a VL domain including CDRL1 including an amino acid sequence represented by SEQ ID NO: 4, CDRL2 including an amino acid sequence represented by SEQ ID NO: 5, and CDRL3 including an amino acid sequence represented by SEQ ID NO: 6.

In an exemplary embodiment, the isolated antibody or the fragment having immunological activity thereof may include a VH domain including an amino acid sequence represented by SEQ ID NO: 7.

In an exemplary embodiment, the isolated antibody or the fragment having immunological activity thereof may include a VL domain including an amino acid sequence represented by SEQ ID NO: 8.

In an exemplary embodiment, the isolated antibody may be a chimeric antibody, a bivalent, a bispecific molecule, a minibody, a domain antibody, a bispecific antibody, an antibody mimic, a diabody, a triabody, or a tetrabody, and the fragment having immunological activity thereof may be any one selected from the group consisting of Fab, Fd, Fd', Fab', dAb, F(ab'), F(ab')2, single chain fragment variable (scFv), Fv, a single-chain antibody, an Fv dimer (dsFv), or a complementarity determining region (CDR) fragment, but is not limited thereto. Preferably, the fragment having immunological activity thereof may be scFv.

In an exemplary embodiment, the antibody of the present disclosure may be a monoclonal antibody.

The antibody includes functional fragments of an antibody molecule as well as a whole antibody form. The whole antibody has a structure having two full-length light chains and two full-length heavy chains, and each light chain is linked to the heavy chain by disulfide bonds. The functional fragment of the antibody molecule refers to a fragment having an antigen binding function. Examples of the antibody fragment include (i) an Fab fragment consisting of a light chain variable region VL, a heavy chain variable region VH, a light chain constant region CL, and a first heavy chain constant region 1 CH1; (ii) an Fd fragment consisting of VH and CH1 domains; (iii) an Fv fragment consisting of VL and VH domains of a single antibody; (iv) a dAb fragment consisting of a VH domain; (v) an isolated CDR region; (vi) an F(ab')2 fragment, which is a bivalent fragment including two linked Fab fragments; (vii) a single-chain Fv molecule (scFv) in which the VH domain and the VL domain are linked to each other by a peptide linker to form an antigen binding domain; (viii) a bispecific single-chain Fv dimer; and (ix) a diabody which is a multivalent or multispecific fragment produced by gene fusion.

The antibody or the fragment having immunological activity thereof of the present disclosure may be selected from the group consisting of animal-derived antibodies, chimeric antibodies, human antibodies, and fragments having immunological activity thereof, but is not limited thereto. In addition, the antibody may be produced recombinantly or synthetically, but is not limited thereto.

The antibody or the fragment having immunological activity thereof may be isolated from a living body (not present in a living body) or may be non-naturally occurring, and for example, may be synthetically or recombinantly produced.

As used herein, the "antibody" refers to a material produced by stimulation of an antigen in the immune system, and the type thereof is not particularly limited, and may be obtained naturally or non-naturally (for example, synthetically or recombinantly). The antibody is very stable *in vivo* as well as *in vitro* and has a long half-life, which is advantageous for mass expression and production. In addition, since the antibody essentially has a dimer structure, its avidity is very high. A complete antibody has a structure having two full-length light chains and two full-length heavy chains, and each light chain is linked to the heavy chain by disulfide bonds. The constant region of the antibody is divided into a heavy chain constant region and a light chain constant region, and the heavy chain constant region has gamma (γ), mu (µ), alpha (α), delta (δ), and epsilon (ε) types, and has subclasses of gamma1 (γ1), gamma2 (γ2), gamma3 (γ3), gamma4 (γ4), alpha1 (α1), and alpha2 (α2). The light chain constant region has kappa (κ) and lambda (λ) types.

As used herein, the term "heavy chain" is interpreted as the meaning including all of a full-length heavy chain including a variable region domain VH including an amino acid sequence having a variable region sequence sufficient for imparting specificity to an antigen, three constant region domains CH1, CH2 and CH3 and hinges, and fragments thereof. In addition, the term "light chain" is interpreted as the meaning including all of a full-length light chain including a variable region domain VL including an amino acid sequence having a variable region sequence sufficient for imparting specificity to an antigen and a constant region domain CL, and fragments thereof.

As used herein, the term "variable region or variable domain" refers to a portion of an antibody molecule that exhibits many variations on the sequence while performing a function that specifically binds to an antigen, and in the variable region, there are complementarity determining regions CDR1, CDR2 and CDR3. A framework region (FR) portion exists between the CDRs to support a CDR ring. The "complementarity determining region" is a ring-shaped region involved in recognition of the antigen, and specificity of the antibody to the antigen is determined as the sequence of this region changes.

As used herein, the term "single chain fragment variable (scFv)" refers to a single-chain antibody formed by expressing only a variable region of the antibody through genetic recombination, and is a single-chain form antibody in which the VH and VL regions of the antibody are linked to each other by a short peptide chain. The term "scFv" is intended to include an scFv fragment including an antigen binding fragment, unless otherwise specified or otherwise understood from the context. This is obvious to those skilled in the art.

As used herein, the term "complementarity determining region (CDR)" refers to an amino acid sequence of a hypervariable region in the heavy and light chains of immunoglobulin. The heavy and light chains may include three CDRs (CDRH1, CDRH2, CDRH3 and CDRL1, CDRL2, CDRL3), respectively. The CDRs may provide key contact residues for the binding of the antibody to an antigen or an epitope.

As used herein, the term "specifically binding" or "specifically recognizing" has the same meaning as commonly known to those skilled in the art, and means that an antigen and an antibody specifically interact with each other to cause an immunological response.

As used herein, the term "antigen binding fragment" refers to a fragment of the entire structure of immunoglobulin, and refers to a portion of a polypeptide including a portion capable of binding to the antigen. For example, the antigen binding fragment may be scFv, (scFv)2, scFv-Fc, Fab, Fab' or F(ab')2, but is not limited thereto. Among the antigen binding fragments, the Fab has a structure having variable regions of the light and heavy chains, a constant region of the light chain, and a first constant region CH1 of the heavy chain, and has one antigen binding domain. The Fab' is different from Fab in that there is a hinge region containing one or more cysteine residues at a C-terminus of the heavy chain CH1 domain.

The F(ab')2 antibody is produced when the cysteine residues in the hinge region of Fab' form disulfide bonds. The Fv is a minimal antibody fragment having only a heavy chain variable region and a light chain variable region, and a recombination technique for producing the Fv fragment is widely known in the art. The two-chain Fv has a heavy chain variable domain and a light chain variable domain linked via a non-covalent bond, and the single-chain Fv may generally form a dimer-like structure, such as the two-chain Fv because the variable region of the heavy chain and the variable region of the single chain are covalently linked via a peptide linker or directly linked at a C-terminus. The linker may be a peptide linker consisting of 1 to 100 or 2 to 50 random amino acids, and appropriate sequences are known in the art. The antigen binding fragments may be obtained using a protease (for example, the whole antibody is restriction-cleaved with papain to obtain Fab, and cleaved with pepsin to obtain an F(ab')2 fragment), and may be produced through genetic recombination technology.

As used herein, the term "hinge region" refers to a region included in the heavy chain of the antibody, and means a region which is present between the CH1 and CH2 regions and functions to provide flexibility of the antigen binding domain in the antibody. For example, the hinge may be derived from a human antibody, and specifically, derived from IgA, IgE, or IgG, such as IgG1, IgG2, IgG3, or IgG4.

In an aspect, the present disclosure provides a humanized antibody specifically binding to GPC3 or an antigen binding fragment thereof including: a human VH domain including any one or more CDRHs selected from the group consisting of complementarity determining regions heavy chain 1 (CDRH1) including an amino acid sequence represented by SEQ ID NO: 1, CDRH2 including an amino acid sequence represented by SEQ ID NO: 2, and CDRH3 including an amino acid sequence represented by SEQ ID NO: 3; and a human VL domain including any one or more CDRLs selected from the group consisting of CDRL1 including an amino acid sequence represented by SEQ ID NO: 4, CDRL2 including an amino acid sequence represented by SEQ ID NO: 5, and CDRL3 including an amino acid sequence represented by SEQ ID NO: 6.

In an exemplary embodiment, the human VH domain may be any one or more selected from the group consisting of VH1 including an amino acid sequence represented by SEQ ID NO: 11; VH2 including an amino acid sequence represented by SEQ ID NO: 12; VH3 including an amino acid sequence represented by SEQ ID NO: 13; and VH4 including an amino acid sequence represented by SEQ ID NO: 14.

In an exemplary embodiment, the human VL domain may be any one or more selected from the group consisting of VL1 including an amino acid sequence represented by SEQ ID NO: 15; VL2 including an amino acid sequence represented by SEQ ID NO: 16; VL3 including an amino acid sequence represented by SEQ ID NO: 17; and VL4 including an amino acid sequence represented by SEQ ID NO: 18.

In an exemplary embodiment, preferably, the humanized antibody may include VH1 including an amino acid sequence represented by SEQ ID NO: 11 and VL1 including an amino acid sequence represented by SEQ ID NO: 15; VH1 including an amino acid sequence represented by SEQ ID NO: 11 and VL2 including an amino acid sequence represented by SEQ ID NO: 16; VH1 including an amino acid sequence represented by SEQ ID NO: 11 and VL3 including an amino acid sequence represented by SEQ ID NO: 17; VH1 including an amino acid sequence represented by SEQ ID NO: 11 and VL4 including an amino acid sequence represented by SEQ ID NO: 18; VH2 including an amino acid sequence represented by SEQ ID NO: 12 and VL1 including an amino acid sequence represented by SEQ ID NO: 15; VH2 including an amino acid sequence represented by SEQ ID NO: 12 and VL2 including an amino acid sequence represented by SEQ ID NO: 16; VH2 including an amino acid sequence represented by SEQ ID NO: 12 and VL3 including an amino acid sequence represented by SEQ ID NO: 17; VH2 including an amino acid sequence represented by SEQ ID NO: 12 and VL4 including an amino acid sequence represented by SEQ ID NO: 18; VH4 including an amino acid sequence represented by SEQ ID NO: 14 and VL3 including an amino acid sequence represented by SEQ ID NO: 17; or VH4 including an amino acid sequence represented by SEQ ID NO: 14 and VL4 including an amino acid sequence represented by SEQ ID NO: 18.

Animal-derived antibodies produced by immunizing an animal to be immunized with a desired antigen may generally cause immune rejection when administered to humans for treatment, and chimeric antibodies have been developed to inhibit such immune rejection. In the chimeric antibody, a constant region of an animal-derived antibody, which causes an anti-isotype response, is substituted with a constant region of a human antibody using a genetic engineering method. Compared to the animal-derived antibodies, the chimeric antibody has been significantly improved in the anti-isotype response, but still includes potential side effects on anti-idiotypic responses because animal-derived amino acids are present in a variable region. The humanized antibody is developed to alleviate these side effects. The humanized antibody is produced by grafting, into a human antibody framework, complementarity determining regions (CDRs), which play an important role in binding to the antigen in the variable region of the chimeric antibody.

In the CDR grafting technology for producing the humanized antibody, it is most important to select an optimized human antibody that may best accept the CDR region of the animal-derived antibody, and to this end, applications of an antibody database, analysis of a crystal structure, molecular modeling technology, and the like are used. However, even if the CDR region of the animal-derived antibody is grafted into the optimized human antibody framework, in some cases, there are amino acids that affect binding to the antigen while being located in the framework of the animal-derived antibody. As a result, since there are many cases in which the antigen binding activity is not conserved, it may be required to apply additional antibody engineering technology for restoring the antigen binding activity.

The antibody or the fragment having immunological activity thereof, or the humanized antibody or the antigen binding fragment thereof of the present disclosure may be prepared by any method known in the art. After encoding a polypeptide sequence of the antibody or the fragment having immunological activity thereof, or the humanized antibody or the antigen binding fragment thereof of the present disclosure, if desired, the polypeptide sequence is cloned into a host cell and used to form a nucleic acid to be expressed and assayed.

A nucleic acid encoding the antibody or the fragment having immunological activity thereof, or the humanized antibody or the antigen binding fragment thereof of the present disclosure may be inserted into an expression vector for protein expression. The expression vector generally includes a protein operably linked, i.e., functionally related with a control or regulatory sequence, a selectable marker, an optional fusion partner, and/or an additional element. Under appropriate conditions, the antibody or the fragment having immunological activity thereof, or the humanized antibody or the antigen binding fragment thereof of the present disclosure may be produced by a method of inducing protein expression by culturing a host cell transformed with a nucleic acid, preferably, an expression vector containing a nucleic acid molecule encoding the antibody or the fragment having immunological activity thereof, or the humanized antibody or the antigen binding fragment thereof according to the present disclosure. A variety of suitable host cells including mammalian cells, bacteria, insect cells, and yeast may be used, but are not limited thereto. Methods for introducing exogenous nucleic acids into host cells are known in the art and will vary depending on a host cell to be used. Preferably, the antibody or the fragment having immunological activity thereof, or the humanized antibody or the antigen binding fragment thereof according to the present disclosure is produced using, as a host cell, *E. coli*, which has low production cost and high industrial utility value.

Therefore, the scope of the present disclosure includes a method for preparing an isolated antibody specifically binding to a GPC3 protein or a fragment having immunological activity thereof, or a humanized antibody or an antigen binding fragment thereof, including: culturing a host cell introduced with a nucleic acid encoding the antibody or the fragment having immunological activity thereof, or the humanized antibody or the antigen binding fragment thereof of the present disclosure under conditions suitable for protein expression; and purifying or isolating the antibody or the fragment having immunological activity thereof expressed from the host cell.

The antibody may be isolated or purified by various methods known in the art. Standard purification methods include chromatographic techniques, electrophoresis, immunoprecipitation, dialysis, filtration, concentration, and chromatofocusing techniques. As known in the art, various natural proteins such as bacterial proteins A, G, and L may bind to the antibodies, and these proteins may be used for purification. The purification may often be enabled by using a particular fusion partner.

In an aspect, the present disclosure relates to an isolated cell producing an antibody or a fragment having immunological activity thereof, or a humanized antibody or an antigen binding fragment thereof of the present disclosure.

In an exemplary embodiment, the cell may be a bacterial cell, a yeast cell, a mammalian cell, an insect cell, or a hybridoma cell, and is most preferably a hybridoma cell line.

In an aspect, the present disclosure relates to an isolated nucleic acid molecule encoding an antibody or a fragment having immunological activity thereof, or a humanized antibody or an antigen binding fragment thereof of the present disclosure, a vector including the same, and a host cell transformed therewith.

In an exemplary embodiment, the nucleic acid molecule encoding the antibody or the fragment having immunological activity thereof may include a nucleotide sequence represented by at least any one selected from the group consisting of SEQ ID NOs: 9 and 10.

The nucleic acid molecule of the present disclosure may be isolated or recombined, and includes not only DNA and RNA in single-chain and double-chain forms, but also corresponding complementary sequences. In the case of a nucleic acid isolated from a naturally occurring source, the isolated nucleic acid is a nucleic acid that is isolated from surrounding genetic sequences present in the genome of a subject from which the nucleic acid is isolated. In the case of a nucleic acid synthesized enzymatically or chemically from a template, such as a PCR product, a cDNA molecule, or an oligonucleotide, the nucleic acid produced from such a procedure may be understood as an isolated nucleic acid molecule. The isolated nucleic acid molecule refers to a nucleic acid molecule in the form of a separate fragment or as a component of a larger nucleic acid construct. The nucleic acid is operably linked when disposed in a functional relationship with another nucleic acid sequence. For example, when being expressed as a preprotein in the form before the polypeptide is secreted, DNA of a pre-sequence or secretory leader is operably linked to DNA of the polypeptide, and a promoter or enhancer is operably linked to a coding sequence when affecting the transcription of the polypeptide sequence, or a ribosome binding domain is operably linked to a coding sequence when disposed to promote the translation. In general, it means that operably linked DNA sequences are located contiguously, and that the secretory leader exists contiguously in the same reading frame. However, the enhancer need not be contiguously located. The linkage is achieved by ligation at a convenient restriction enzyme site. When there is no site, synthetic oligonucleotide adapters or linkers are used according to conventional methods.

In the isolated nucleic acid molecule encoding the antibody or the fragment having immunological activity thereof, or the humanized antibody or the antigen binding fragment thereof of the present disclosure, due to the degeneracy of codons or in consideration of codons preferred in an organism in which the antibody is to be expressed, it will be well understood to those skilled in the art that various modifications may be made in a coding region within a range without changing the amino acid sequence of the antibody to be expressed from the coding region, various modifications or changes may be made within a range without affecting the expression of the gene even in parts other than the coding region, and such modified genes are also included within the scope of the present disclosure. That is, as long as the nucleic acid molecule of the present disclosure encodes a protein having equivalent activity thereto, one or more nucleobases may be mutated by substitution, deletion, insertion, or a combination thereof, which are also included in the scope of the present disclosure. The sequence of such a nucleic acid molecule may be single- or double-chain, and may be a DNA molecule or an RNA (mRNA) molecule.

The isolated nucleic acid molecule encoding the antibody or the fragment having immunological activity thereof, or the humanized antibody or the antigen binding fragment thereof according to the present disclosure may be inserted into an expression vector for protein expression. The expression vector generally includes a protein operably linked, i.e., functionally related with a control or regulatory sequence, a selectable marker, an optional fusion partner, and/or an additional element. Under appropriate conditions, the antibody or the fragment having immunological activity thereof, or the humanized antibody or the antigen binding fragment thereof of the present disclosure may be produced by a method of inducing protein expression by culturing a host cell transformed with a nucleic acid, preferably, an isolated expression vector containing a nucleic acid molecule encoding the antibody or the fragment having immunological activity thereof, or the humanized antibody or the antigen binding fragment thereof of the present disclosure. A variety of suitable host cells including mammalian cells, bacteria, insect cells, and yeast may be used, but are not limited thereto. Methods for introducing exogenous nucleic acids into host cells are known in the art and will vary depending on a host cell to be used. Preferably, it is possible to produce *E. coli*, which has high industrial value due to low production cost, as a host cell.

The vector of the present disclosure may include a plasmid vector, a cosmid vector, a bacteriophage vector, a viral vector, etc., but is not limited thereto. The suitable vector includes a signal sequence or a leader sequence for membrane targeting or secretion in addition to expression regulatory elements such as a promoter, an operator, an initiation codon, a termination codon, a polyadenylation signal, and an enhancer, and may be variously produced according to a purpose. The promoter of the vector may be constitutive or inducible. The signal sequence may use a PhoA signal sequence, an OmpA signal sequence, etc. in the case of *Escherichia* sp. bacteria as a host, an α-amylase signal sequence, a subtilisin signal sequence, etc. in the case of *Bacillus* sp. bacteria as a host, an MFα signal sequence, an SUC2 signal sequence, etc. in the case of yeast as a host, and an insulin signal sequence, an α-interferon signal sequence, an antibody molecule signal sequence, etc. in the case of an animal cell as a host, but is not limited thereto. Further, the vector may include a selectable marker for selecting a host cell containing a vector and a replicable expression vector includes a replication origin.

As used herein, the term "vector" refers to a vehicle into which a nucleic acid sequence may be inserted for introduction into a cell capable of replicating the nucleic acid sequence. The nucleic acid sequence may be exogenous or heterologous. The vector may include plasmids, cosmids, and viruses (for example, bacteriophage), but is not limited thereto.

Those skilled in the art may construct vectors by standard recombinant techniques.

In an exemplary embodiment, when constructing the vector, expression regulatory sequences such as a promoter, a terminator, and an enhancer, sequences for membrane targeting or secretion, etc. may be appropriately selected according to a type of host cell intended to produce the antibody and may be variously combined depending on a purpose.

As used herein, the term "expression vector" refers to a vector including a nucleic acid sequence encoding at least a portion of a gene product to be transcribed. In some cases, an RNA molecule is then translated into a protein, a polypeptide, or a peptide. The expression vector may include various regulatory sequences. In addition to regulatory sequences that regulate transcription and translation, vectors and expression vectors may also include nucleic acid sequences that provide other functions.

As used herein, the term "host cell" includes eukaryotes and prokaryotes, and refers to any transformable organism capable of replicating the vector or expressing a gene encoded by the vector. The host cell may be transfected or transformed by the vector, which means a process in which an exogenous nucleic acid molecule is delivered or introduced into the host cell.

In an exemplary embodiment, the host cell may be a bacterial cell, a yeast cell, a mammalian cell, an insect cell, or a hybridoma cell, and may be a CHO cell, a HEK 293 cell, a CAP cell, a Per.C6 cell, a BHK cell, a Vero cell, an MDCK cell, a hybridoma cell, or a fibroblast.

In an aspect, the present disclosure relates to a composition for detecting cancer antigens including an antibody or a fragment having immunological activity thereof according to the present disclosure, or a humanized antibody or an antigen binding fragment thereof according to the present disclosure.

In an exemplary embodiment, the cancer antigen may be GPC3.

As used herein, the term "detection" refers to the detection of an antigen-antibody conjugate and may be carried out using various markers. Although not limited thereto, specific examples of the marker include enzymes, fluorescent substances, ligands, luminescent substances, microparticles, or radioactive isotopes.

Enzymes used as detection markers include acetylcholinesterase, alkaline phosphatase, β-D-galactosidase, horseradish peroxidase, β-latamase, etc., fluorescent substances include fluorescein, Eu3+, Eu3+ chelate or cryptate, etc., ligands include biotin derivatives, etc., luminescent substances include acridinium ester, isoluminol derivatives, etc., and microparticles include colloidal gold, colored latex, etc., and radioactive isotopes include 57Co, 3H, 125I, and 125I-Bonton Hunter reagents, etc.

The detection composition of the present disclosure may detect antigen-antibody conjugates using an enzyme-linked immunosorbent assay (ELISA). The ELISA includes various ELISA methods, such as direct ELISA using a labeled antibody that recognizes an antigen attached to a solid support, indirect ELISA using a labeled secondary antibody that recognizes a capture antibody in a conjugate of an antibody that recognizes an antigen attached to a solid support, direct sandwich ELISA using another labeled antibody that recognizes an antigen in a conjugate of an antibody and an antigen attached to a solid support, indirect sandwich ELISA using a labeled secondary antibody that recognizes an antibody after reacting with another antibody that recognizes the antigen in a conjugate of the antibody and the antigen attached to a solid support, and the like. The antibody according to the present disclosure may have a detection label, and if the antibody does not have a detection label, these monoclonal antibodies may be captured and confirmed by treatment with another antibody having a detection label.

In addition, 'detection' as used herein may also be performed using a label-free method that does not use a marker. Specifically, the label-free method is selected from surface plasmon resonance, isothermal titration calorimetry, and bio-layer interferometry. However, it is not necessarily limited to these.

In an aspect, the present disclosure relates to a kit for detecting cancer antigens including an antibody or a fragment having immunological activity thereof according to the present disclosure, or a humanized antibody or an antigen binding fragment thereof according to the present disclosure.

In an exemplary embodiment, the cancer antigen may be GPC3.

The kit for detecting cancer antigens of the present disclosure may include an antibody or a fragment having immunological activity thereof according to the present disclosure, or a humanized antibody or an antigen binding fragment thereof according to the present disclosure, and tools and reagents used for immunological analysis. The tools and reagents used in immunological analysis include suitable carriers, labeling substances capable of generating detectable signals, solubilizers, and cleaning agents. In addition, when the labeling substance is an enzyme, the kit may include a substrate for measuring enzyme activity and a reaction stopper.

Suitable carriers may include, but are not limited to, soluble carriers, for example, physiologically acceptable buffers known in the art, such as PBS; insoluble carriers, for example, polymers such as polystyrene, polyethylene, polypropylene, polyester, polyacrylonitrile, fluoropolymer, cross-linked dextran, polysaccharide, and magnetic microparticles plated with metal on a latex, other paper, glass, metal, agarose, and combinations thereof.

An assay system for use in the detection composition or kit of the present disclosure includes, but is not limited to, ELISA plates, dip-stick devices, immunochromatography test strips and radiosegmented immunoassay devices, flow-through devices, etc.

In an aspect, the present disclosure relates to an antibody-drug conjugate (ADC) including an isolated antibody or a fragment having immunological activity thereof of the present disclosure, or a humanized antibody or an antigen binding fragment thereof of the present disclosure; and a drug.

In an exemplary embodiment, the drug may be an immunogenic apoptosis inducer, a microtubulin structure formation inhibitor, a meiosis inhibitor, a topoisomerase inhibitor, a DNA intercalator, a toxin, or a radionuclide. The immunogenic apoptosis inducer may be any one or more selected from the group consisting of anthracycline-based anticancer agents, taxane-based anticancer agents, anti-EGFR antibodies, BK channel agonists, bortezomib, cardiac glycoside, cyclophosphamide-based anticancer agents, GADD34/PP1 inhibitor, LV-tSMAC, Measles virus, bleomycin, mitoxantrone, or oxaliplatin, but is not limited thereto.

In an exemplary embodiment, the drug may be selected from the group consisting of 7-ethyl-10-hydroxy-camptothecin (SN-38), daunorubicin, doxorubicin, epirubicin, idarubicin, pixantrone, sabarubicin, valrubicin, paclitaxel, docetaxel, mechloethamine, chlorambucil, phenylalanine, mustard, cyclophosphamide, ifosfamide, carmustine (BCNU), lomustine (CCNU), streptozotocin, busulfan, thiotepa, cisplatin, carboplatin, dactinomycin (actinomycin D), plicamycin, mitomycin C, vincristine, vinblastine, teniposide, topotecan, iridotecan, uramustine, melphalan, bendamustine, dacarbazine, temozolomide, altretamine, duocarmycin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, etoposide, mitoxantrone, izabepilone, vindesine, vinorelbine, estramustine, maytansine, mertansine (DM1), DM4, dolastatin, auristatin E, auristatin F, monomethyl auristatin E (MMAE), monomethyl auristatin F, and derivatives thereof, but is not limited thereto.

In an exemplary embodiment, the antibody-drug conjugate may further include an ADC linker, and the ADC linker may be 6-maleimidocaproyl (MC), maleimidopropanoyl (MP), valine-citrulline (val-cit), alanine-phenylalanine (ala-phe), p-aminobenzyloxycarbonyl (PAB), N-succinimidyl 4-(2-pyridylthio) pentanoate (SPP), N-succinimidyl 4-(N-maleimidomethyl) cyclohexane-1 carboxylate (SMCC), valine-citrulline-p-aminobenzyloxycarbonyl (val-cit-PAB), or N-succinimidyl (4-iodo-acetyl) aminobenzoate (SIAB), but is not limited thereto.

In an exemplary embodiment, the antibody-drug conjugate may form a conjugate with the drug and the antibody or the fragment having immunological activity thereof of the present disclosure via an ADC linker.

In an aspect, the present disclosure relates to a pharmaceutical composition for preventing or treating cancer including an antibody-drug conjugate, including an isolated antibody or a fragment having immunological activity thereof of the present disclosure, or a humanized antibody or an antigen binding fragment thereof of the present disclosure; and a drug.

In an exemplary embodiment, the cancer may be refractory to an immune checkpoint inhibitor.

In an exemplary embodiment, the cancer may be at least any one selected from the group consisting of brain tumor, melanoma, myeloma, non-small cell lung cancer, oral cancer, liver cancer, stomach cancer, colon cancer, breast cancer, triple negative breast cancer (TNBC), lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph node cancer, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or ureteral cancer, renal cell carcinoma, renal pelvic carcinoma, central nervous system tumor, primary central nervous system lymphoma, spinal cord tumor, brainstem glioma and pituitary adenoma, but is not limited thereto.

The pharmaceutical composition for preventing or treating cancer of the present disclosure is administered together with a chemical anticancer drug (anticancer agent) and the like to increase a cancer treatment effect of conventional anticancer agents through a cancer cell killing effect. Combined administration may be performed simultaneously or sequentially with the anticancer agent. The anticancer agent may be one or more selected from the group consisting of cytotoxic anticancer agents, targeted anticancer agents, and cancer immunotherapies, but is not limited thereto. The cytotoxic anticancer agent may be doxorubicin, cyclophosphamide, temozolomide, irinotecan, cisplatin, vinblastine, vincristine, actinomycin-D, 5-fluouracil, docetaxel, cabazitaxel, paclitaxel, or pembrolizumab; the targeted anticancer drug may be Trametinib, Vemurafenib, Alpelisib, Dactolisib, Gefitinib, Erlotinib, Lapatinib, Sunitinib, Sorafenib, Crizotinib, Dabrafenib, Trastuzumab, Cetuximab, Bevacizumab, Panitumumab, Ipilimumab, Pertuzumab, Tofacitinib, Imatinib, Bortezomib, Ofatumumab, or Alemtuzumab; and the cancer immunotherapy may be anti-PD-1, anti-PD-L1, anti-CTLA-4, anti-LAG-3, anti-TIGIT, anti-TIM-3, anti-GITR, CAR-T, or NK cell.

In addition, the anticancer agent may include DNA alkylating agents such as mechloethamine, chlorambucil, phenylalanine, mustard, cyclophosphamide, ifosfamide, carmustine (BCNU), lomustine (CCNU), streptozotocin, busulfan, thiotepa, cisplatin, and carboplatin; anti-cancer antibiotics such as dactinomycin (actinomycin D), plicamycin, and mitomycin C; plant alkaloids such as vincristine, vinblastine, etoposide, teniposide, topotecan, and iridotecan; and the like, but is not limited thereto.

As used herein, the term "prevention" means all actions that inhibit or delay the occurrence, spread, and recurrence of cancer by administration of the pharmaceutical composition according to the present disclosure.

As used herein, the term "treatment" refers to all actions that kill cancer cells, or alleviate or beneficially change the symptoms of cancer by administration of the composition of the present disclosure. Those skilled in the art to which the present disclosure pertains will identify the exact criteria of a disease in which the composition of the present disclosure is effective and determine the degree of alleviation, improvement and treatment with reference to data presented by the Korean Medical Association, etc.

As used herein, the term "therapeutically effective amount" means an amount of pharmaceutically acceptable salt of the composition effective for preventing or treating a target disease, and the therapeutically effective amount of the composition of the present disclosure may vary depending on many factors, such as a method of administration, a target site, the condition of a patient, and the like. Accordingly, when used in the human body, a dose should be determined in an appropriate amount in consideration of safety and efficiency.

It is also possible to estimate the amount used in humans from the effective amount determined by animal experiments.

The pharmaceutical composition of the present disclosure is administered in a pharmaceutically effective amount. As used herein, the term "pharmaceutically effective amount" refers to an amount sufficient to treat a disease at a reasonable benefit/risk ratio applicable to medical treatment and enough not to cause side effects. The effective dose level may be determined according to factors including the health condition of a patient, the type and severity of cancer, the activity of a drug, the sensitivity to a drug, a method of administration, a time of administration, a route of administration, an excretion rate, duration of treatment, and drugs used in combination or simultaneously, and other factors well-known in the medical field. The composition of the present disclosure may be administered as an individual therapeutic agent or in combination with other therapeutic agents, and may be administered sequentially or simultaneously with conventional therapeutic agents, and administered in a single or multiple doses. In consideration of all of the factors, it is important to administer the pharmaceutical composition in an amount capable of obtaining a maximum effect with a minimal amount without side effects, which may be easily determined by those skilled in the art.

The pharmaceutical composition of the present disclosure may further include pharmaceutically acceptable additives. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, arabic gum, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, white sugar, dextrose, sorbitol, talc, and the like. The pharmaceutically acceptable additive according to the present disclosure is preferably included in an amount of 0.1 part by weight to 90 parts by weight based on the composition, but is not limited thereto.

The composition of the present disclosure may also include carriers, diluents, excipients, or combinations of two or more thereof, which are commonly used in biological agents. The pharmaceutically acceptable carrier is not particularly limited as long as the pharmaceutically acceptable carrier is suitable for *in vivo* delivery of the composition, and may be used by combining, for example, compounds, saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be formulated in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be preferably formulated according to each disease or ingredient by an appropriate method in the art.

The composition of the present disclosure may be administered parenterally (e.g., applied with injectable formulations intravenously, subcutaneously, intraperitoneally or topically) or orally according to a desired method, and the range of the dose may vary depending on the body weight, age, sex, and health condition of a patient, a diet, an administration time, an administration method, an excretion rate, the severity of a disease, and the like. A daily dose of the composition according to the present disclosure is 0.0001 to 10 mg/ml, preferably 0.0001 to 5 mg/ml, and more preferably administered once to several times a day.

Liquid formulations for oral administration of the composition of the present disclosure correspond to suspensions, oral liquids, emulsions, syrups, etc., and may include various excipients, such as a wetting agent, a sweetener, a flavor, a preservative, and the like, in addition to water and liquid paraffin, which are simple diluents commonly used. Formulations for parenteral administration include sterilized aqueous solutions, non-aqueous solvents, suspensions, emulsions, freeze-dried formulations, suppositories, and the like.

In an aspect, the present disclosure provides a composition for diagnosing cancer including an isolated antibody or a fragment having immunological activity thereof of the present disclosure, a humanized antibody or an antigen binding fragment thereof of the present disclosure, or an antibody-drug conjugate of the present disclosure.

In an aspect, the present disclosure provides a kit for diagnosing cancer including an isolated antibody or a fragment having immunological activity thereof of the present disclosure, a humanized antibody or an antigen binding fragment thereof of the present disclosure, or an antibody-drug conjugate of the present disclosure.

In the present disclosure, the specific description of the composition for diagnosing cancer and the kit for diagnosing cancer is the same as that described in the aforementioned composition for detecting cancer antigens and the kit for detecting cancer antigens.

In an aspect, the present disclosure provides a method for providing information for the diagnosis of cancer including: treating a sample with an isolated antibody or a fragment having immunological activity thereof of the present disclosure, a humanized antibody or an antigen binding fragment thereof of the present disclosure, or an antibody-drug conjugate of the present disclosure, which is labeled with a fluorescent substance; and measuring the expression of the isolated antibody or the fragment having immunological activity thereof, the humanized antibody or the antigen binding fragment thereof, or the antibody-drug conjugate.

The fluorescent substance has the same meaning as the "marker" of the composition for detecting cancer antigens, and the description thereof is also the same.

In an exemplary embodiment, when the expression of the isolated antibody or the fragment having immunological activity thereof, the humanized antibody or the antigen binding fragment thereof, or the antibody-drug conjugate is measured, it may be diagnosed as having developed cancer.

In the present disclosure, the "sample" is used in the broadest sense. Meanwhile, the sample includes specimens or cultures (e.g., microorganism cultures). On the other hand, the sample means including both biological and environmental samples. In addition, the sample may include synthetic-origin specimens. The biological sample may be at least any one selected from the group consisting of whole blood, plasma, serum, sputum, tears, mucus, nasal washes, nasal aspirate, breath, urine, semen, saliva, peritoneal washings, ascites, cystic fluid, meningeal fluid, amniotic fluid, leukocytes, peripheral blood mononuclear cells, buffy coat, glandular fluid, pancreatic fluid, lymph fluid, pleural fluid, nipple aspirate, bronchial aspirate, synovial fluid, joint aspirate, organ secretions, cells, cell extract, and cerebrospinal fluid, but is not limited thereto.

In an aspect, the present disclosure relates to a method for preparing an isolated antibody specifically binding to a GPC3 protein or a fragment having immunological activity thereof, or a humanized antibody or an antigen binding activity thereof including: culturing a hybridoma cell line that produces the isolated antibody or the fragment having immunological activity thereof of the present disclosure, or the humanized antibody or the antigen binding fragment thereof of the present disclosure.

In an aspect, the present disclosure relates to a method for preparing an isolated antibody specifically binding to a GPC3 protein or a fragment having immunological activity thereof, or a humanized antibody or an antigen binding fragment thereof, including: culturing a host cell transformed with a vector including an isolated nucleic acid molecule encoding an isolated antibody or a fragment having immunological activity thereof, or a humanized antibody or an antigen binding fragment thereof of the present disclosure; and recovering the antibody or the fragment having immunological activity thereof from the host cell culture.

In an aspect, the present disclosure relates to a GPC3-specific chimeric antigen receptor (CAR) including, as an antigen binding domain, a single chain variable fragment (scFv) including a heavy chain variable region including complementarity determining regions heavy chain 1 (CDRH1) including an amino acid sequence represented by SEQ ID NO: 1, CDRH2 including an amino acid sequence represented by SEQ ID NO: 2, and CDRH3 including an amino acid sequence represented by SEQ ID NO: 3; and a light chain variable region including CDRL1 including an amino acid sequence represented by SEQ ID NO: 4, CDRL2 including an amino acid sequence represented by SEQ ID NO: 5, and CDRL3 including an amino acid sequence represented by SEQ ID NO: 6.

In an exemplary embodiment, the light chain variable region may include an amino acid sequence represented by SEQ ID NO: 19.

In an exemplary embodiment, the heavy chain variable region may include an amino acid sequence represented by SEQ ID NO: 20.

In an exemplary embodiment, the heavy chain variable region and the light chain variable region may be linked with each other via a GS linker. The GS linker may include an amino acid sequence represented by SEQ ID NO: 21.

Accordingly, in an exemplary embodiment, the GPC3-specific chimeric antigen receptor of the present disclosure, in which the light chain variable region including the amino acid sequence represented by SEQ ID NO: 19 and the heavy chain variable region including the amino acid sequence represented by SEQ ID NO: 20 are linked with each other via the GS linker including the amino acid sequence represented by SEQ ID NO: 21, may include an scFv including an amino acid sequence represented by SEQ ID NO: 22.

In an aspect, the present disclosure provides a GPC3-specific humanized antibody-derived chimeric antigen receptor (CAR) including: as an antigen binding domain, a single chain variable fragment (scFv) including any one heavy chain variable region selected from the group consisting of a heavy chain variable region including an amino acid sequence represented by SEQ ID NO: 11; a heavy chain variable region including an amino acid sequence represented by SEQ ID NO: 12; a heavy chain variable region including an amino acid sequence represented by SEQ ID NO: 13; and a heavy chain variable region including an amino acid sequence represented by SEQ ID NO: 14; and any one light chain variable region selected from the group consisting of a light chain variable region including an amino acid sequence represented by SEQ ID NO: 15; a light chain variable region including an amino acid sequence represented by SEQ ID NO: 16; a light chain variable region including an amino acid sequence represented by SEQ ID NO: 17; and a light chain variable region including an amino acid sequence represented by SEQ ID NO: 18.

In an exemplary embodiment, the scFv may include any one selected from the group consisting of a heavy chain variable region including an amino acid sequence represented by SEQ ID NO: 11 and a light chain variable region including an amino acid sequence represented by SEQ ID NO: 15; a heavy chain variable region including an amino acid sequence represented by SEQ ID NO: 11 and a light chain variable region including an amino acid sequence represented by SEQ ID NO: 17; a heavy chain variable region including an amino acid sequence represented by SEQ ID NO: 12 and a light chain variable region including an amino acid sequence represented by SEQ ID NO: 16; and a heavy chain variable region including an amino acid sequence represented by SEQ ID NO: 12 and a light chain variable region including an amino acid sequence represented by SEQ ID NO: 18.

In an exemplary embodiment, the heavy chain variable region and the light chain variable region may be linked with each other via a GS linker. The GS linker may include an amino acid sequence represented by SEQ ID NO: 21.

Accordingly, in an exemplary embodiment, the GPC3-specific humanized antibody-derived chimeric antigen receptor of the present disclosure, in which the heavy chain variable region including the amino acid sequence represented by SEQ ID NO: 11 and the light chain variable region including the amino acid sequence represented by SEQ ID NO: 15 are linked with each other via the GS linker including the amino acid sequence represented by SEQ ID NO: 21, may include an amino acid sequence represented by SEQ ID NO: 23.

In addition, in an exemplary embodiment, the GPC3-specific humanized antibody-derived chimeric antigen receptor of the present disclosure, in which the heavy chain variable region including the amino acid sequence represented by SEQ ID NO: 11 and the light chain variable region including the amino acid sequence represented by SEQ ID NO: 17 are linked with each other via the GS linker including an amino acid sequence represented by SEQ ID NO: 21, may include an amino acid sequence represented by SEQ ID NO: 24.

In addition, in an exemplary embodiment, the GPC3-specific humanized antibody-derived chimeric antigen receptor of the present disclosure, in which the heavy chain variable region including the amino acid sequence represented by SEQ ID NO: 12 and the light chain variable region including the amino acid sequence represented by SEQ ID NO: 16 are linked with each other via the GS linker including the amino acid sequence represented by SEQ ID NO: 21, may include an amino acid sequence represented by SEQ ID NO: 25.

In addition, in an exemplary embodiment, the GPC3-specific humanized antibody-derived chimeric antigen receptor of the present disclosure, in which the heavy chain variable region including the amino acid sequence represented by SEQ ID NO: 12 and the light chain variable region including the amino acid sequence represented by SEQ ID NO: 18 are linked with each other via the GS linker including the amino acid sequence represented by SEQ ID NO: 21, may include an amino acid sequence represented by SEQ ID NO: 26.

The antibody, the humanized antibody, the GPC3-specific chimeric antigen receptor, or the GPC3-specific humanized antibody-derived CAR of the present disclosure may include variants of amino acid sequences described in the attached sequence list within a range capable of specifically recognizing GPC3. For example, changes in the amino acid sequence of the antibody may be made to improve the binding affinity and/or other biological properties of the antibody. These modifications include, for example, deletion, insertion and/or substitution of amino acid sequence residues of the antibody.

The amino acid variant is made based on the relative similarity of an amino acid side-chain substituent, such as hydrophobicity, hydrophilicity, charges, sizes and the like. By analysis of the size, shape and type of the amino acid side-chain substituent, it can be seen that arginine, lysine and histidine are all positively charged residues; alanine, glycine and serine have similar sizes; and phenylalanine, tryptophan and tyrosine have similar shapes. Accordingly, based on these considerations, arginine, lysine and histidine; alanine, glycine and serine; and phenylalanine, tryptophan and tyrosine may be biologically functional equivalents.

In introducing a mutation, a hydrophobic index of amino acids may be considered. Each amino acid is assigned with a hydrophobic index according to its hydrophobicity and charges: isoleucine (+4.5); valine (+4.2); leucine (+3.8); phenylalanine (+2.8); cysteine/cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The hydrophobic amino acid index is very important in assigning an interactive biological function of the proteins. It is known that only substitution with amino acids having similar hydrophobic indices may retain similar biological activity. When introducing mutations with reference to the hydrophobic index, substitution is made between amino acids which exhibit a hydrophobic index difference, preferably within ± 2, more preferably within ± 1, and much more preferably within ± 0.5.

On the other hand, it is also well known that substitutions between amino acids having similar hydrophilicity values result in proteins having equivalent biological activity. The following hydrophilicity values are assigned to each amino acid residue: arginine (+3.0); lysine (+3.0); aspartate (+ 3.0 ± 1); glutamate (+ 3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

When introducing mutations with reference to hydrophilicity values, substitution is made between amino acids which exhibit a hydrophilicity value difference, preferably within ± 2, more preferably within ± 1, and much more preferably within ± 0.5.

In proteins that do not change the overall activity of the molecule, the most commonly occurring exchange is an exchange between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly.

As used herein, the term "chimeric antigen receptor (CAR)" refers to a non-naturally occurring receptor capable of imparting specificity for a specific antigen to an immune effector cell. Typically, the CAR refers to a receptor used to graft the specificity of a monoclonal antibody into a T cell. In addition, the GPC3-specific chimeric antigen receptor or the GPC3-specific humanized antibody-derived chimeric antigen receptor of the present disclosure may further include one or more selected from the group consisting of a signal peptide, a stem domain, a transmembrane domain, and an intracellular signaling domain.

In the present disclosure, the signal peptide (signal sequence) serves to send an initial protein to the endoplasmic reticulum, and any polypeptide known to have the same or similar function as described above may be used without restriction, regardless of a natural or synthetic origin thereof. Although not limited thereto, the signal peptide may be one or more selected from the group consisting of CD8, CD28, GM-CSF, CD4 and CD137.

As used herein, the term "stem domain" means any polypeptide that functions to connect a transmembrane domain to scFv of the present disclosure. Any polypeptide known to perform the same or similar functions as described above may be used without restriction, regardless of a natural or synthetic origin thereof. Although not limited thereto, the stem domain may be one selected from the group consisting of IgG1, IgG2, IgG3, IgG4, IgA, IgD, IgE, IgM, CD28, and CD8 hinges.

As used herein, the term "transmembrane domain" refers to any polypeptide that causes the CAR according to the present disclosure to be expressed on the surface membrane of a cell. The transmembrane domain suitable for the CAR disclosed in the present disclosure has (a) the ability to be expressed on the surface of a cell, for example, an immune cell, e.g., unrestrictedly a lymphocyte cell or a natural killer (NK) cell, and (b) the ability to interact with scFv according to the present disclosure and an intracellular signaling domain to direct a cellular response of the immune cell toward a predetermined target cell. As the transmembrane domain, any polypeptide known to have the same or similar functions as described above may also be used without restriction, regardless of a natural or synthetic origin thereof. Although not limited thereto, the transmembrane domain may be at least one selected from the group consisting of FcγR, ICOS(CD278), 4-1BB(CD137), OX40(CD134), CD27, CD28, IL-2Rβ, CD40, DAP10, MHC class I molecule, TNF receptor protein, Immunoglobulin-like protein, cytokine receptor, integrin, SLAM protein, activated NK cell receptor, BTLA, Toll ligand receptor, CD2, CD7, CD30, CDS, ICAM-1, B7-H3, GITR, BAFFR, LIGHT, HVEM (LIGHTR), KIRDS2, SLAMF7, NKp80 (KLRF1), NKp44, NKp30, NKp46, CD19, CD4, CD8α, CD8β, ITGA4, VLA1, CD49a, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD83 specific ligand, CD247, CD3δ, CD3ε, CD3γ, CD3ζ, CD8, Ig alpha(CD79a), IL-2Rγ, IL-7Rα, PD-1, TNFSF14, CD45, CD5, CD9, CD16, CD22, CD33, CD37, CD64, CD80, CD86, CD154, an alpha chain of a T cell receptor, a beta chain of a T cell receptor, and a zeta chain of a T cell receptor.

As used herein, the term "intracellular signaling domain" refers to a portion of a protein that transmits an effector signal functional signal and directs the cell to perform a specialized function. After the scFv according to the present disclosure binds to a target, the intracellular signaling domain is responsible for intracellular signaling to cause T cell activation. As the intracellular signaling domain, any polypeptide known to have the same or similar functions as described above may also be used without restriction, regardless of a natural or synthetic origin thereof.

Although not limited thereto, the intracellular signaling domain may be at least any one selected from the group consisting of CD3ζ, FcyR, ICOS(CD278), 4-1BB(CD137), OX40(CD134), CD27, CD28, IL-2Rβ, IL-15R-α, MyD88, DAP10, DAP12, MHC class I molecule, TNF receptor protein, Immunoglobulin-like protein, cytokine receptor, integrin, SLAM protein, activated NK cell receptor, BTLA, Ttoll ligand receptor, CD2, CD7, CD30, CD40, CDS, ICAM-1, B7-H3, GITR, BAFFR, LIGHT, HVEM (LIGHTR), KIRDS2, SLAMF7, NKp80 (KLRF1), NKp44, NKp30, NKp46, CD19, CD4, CD8α, CD8β, IL2Rγ, IL7Rα, ITGA4, VLA1, CD49a, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD83 specific ligands, CD247, CD3δ, CD3ε, CD3γ, CD8, Ig alpha (CD79a), IL-2Rγ, IL-7Rα, PD-1, and TNFSF14.

The GPC3-specific chimeric antigen receptor or the GPC3-specific humanized antibody-derived chimeric antigen receptor of the present disclosure may further include a tag peptide capable of confirming the expression thereof. Any known tag peptide, such as a flag peptide, may be used without limitation.

In an exemplary embodiment, a GPC3-specific CAR in which a CD8 signal peptide, a FLAG tag, a light chain variable region including an amino acid sequence represented by SEQ ID NO: 19, a GS linker represented by SEQ ID NO: 21, a heavy chain variable region including an amino acid sequence represented by SEQ ID NO: 20, and CD28 and CD3ζ are sequentially linked, includes an amino acid sequence represented by SEQ ID NO: 28.

In an exemplary embodiment, a GPC3-specific CAR in which a CD8 signal peptide, a FLAG tag, a light chain variable region including an amino acid sequence represented by SEQ ID NO: 19, a GS linker represented by SEQ ID NO: 21, a heavy chain variable region including an amino acid sequence represented by SEQ ID NO: 20, a CD8 transmembrane domain, 4-1BB and CD3ζ are sequentially linked, includes an amino acid sequence represented by SEQ ID NO: 29.

In an exemplary embodiment, a GPC3-specific humanized antibody-derived CAR in which a CD8 signal peptide, a FLAG tag, a light chain variable region including an amino acid sequence represented by SEQ ID NO: 15, a GS linker represented by SEQ ID NO: 21, a heavy chain variable region including an amino acid sequence represented by SEQ ID NO: 11, CD28 and CD3ζ are sequentially linked, includes an amino acid sequence represented by SEQ ID NO: 30.

In an exemplary embodiment, a GPC3-specific humanized antibody-derived CAR in which a CD8 signal peptide, a FLAG tag, a light chain variable region including an amino acid sequence represented by SEQ ID NO: 17, a GS linker represented by SEQ ID NO: 21, a heavy chain variable region including an amino acid sequence represented by SEQ ID NO: 11, CD28 and CD3ζ are sequentially linked, includes an amino acid sequence represented by SEQ ID NO: 31.

In an exemplary embodiment, a GPC3-specific humanized antibody-derived CAR in which a CD8 signal peptide, a FLAG tag, a light chain variable region including an amino acid sequence represented by SEQ ID NO: 16, a GS linker represented by SEQ ID NO: 21, a heavy chain variable region including an amino acid sequence represented by SEQ ID NO: 12, CD28 and CD3ζ are sequentially linked, includes an amino acid sequence represented by SEQ ID NO: 32.

In an exemplary embodiment, a GPC3-specific humanized antibody-derived CAR in which a CD8 signal peptide, a FLAG tag, a light chain variable region including an amino acid sequence represented by SEQ ID NO: 18, a GS linker represented by SEQ ID NO: 21, a heavy chain variable region including an amino acid sequence represented by SEQ ID NO: 12, CD28 and CD3ζ are sequentially linked, includes an amino acid sequence represented by SEQ ID NO: 33.

In an aspect, the present disclosure provides a polynucleotide including a nucleic acid sequence encoding a GPC3-specific CAR according to the present disclosure or a GPC3-specific humanized antibody-derived CAR according to the present disclosure.

The nucleic acid molecule of the present disclosure may be isolated or recombined, and includes not only DNA and RNA in single-chain and double-chain forms, but also corresponding complementary sequences. In the case of a nucleic acid isolated from a naturally occurring source, the isolated nucleic acid is a nucleic acid that is isolated from surrounding genetic sequences present in the genome of a subject from which the nucleic acid is isolated. In the case of a nucleic acid synthesized enzymatically or chemically from a template, such as a PCR product, a cDNA molecule, or an oligonucleotide, the nucleic acid produced from such a procedure may be understood as an isolated nucleic acid molecule. The isolated nucleic acid molecule refers to a nucleic acid molecule in the form of a separate fragment or as a component of a larger nucleic acid construct. The nucleic acid is operably linked when disposed in a functional relationship with another nucleic acid sequence. For example, when expressed as a preprotein in the form before the polypeptide is secreted, DNA of a pre-sequence or secretory leader is operably linked to DNA of the polypeptide, a promoter or enhancer is operably linked to a coding sequence when affecting the transcription of the polypeptide sequence, or a ribosome binding domain is operably linked to a coding sequence when disposed to promote the translation. In general, it means that operably linked DNA sequences are located contiguously, and that the secretory leader exists contiguously in the same reading frame. However, the enhancer need not be contiguously located. The linkage is achieved by ligation at a convenient restriction enzyme site. When there is no site, synthetic oligonucleotide adapters or linkers are used according to conventional methods.

In the polynucleotide encoding the GPC3-specific CAR of the present disclosure or the GPC3-specific humanized antibody-derived CAR of the present disclosure, due to the degeneracy of codons or in consideration of codons preferred in an organism in which the antibody is to be expressed, it will be well understood to those skilled in the art that various modifications may be made in a coding region within a range without changing the amino acid sequence of the antibody to be expressed from the coding region, various modifications or changes may be made within a range without affecting the expression of the gene even in parts other than the coding region, and such modified genes are also included within the scope of the present disclosure. That is, as long as the nucleic acid molecule of the present disclosure encodes a protein having equivalent activity thereto, one or more nucleobases may be mutated by substitution, deletion, insertion, or a combination thereof, which are also included in the scope of the present disclosure. The sequence of such a nucleic acid molecule may be single- or double-chain, and may be a DNA molecule or an RNA (mRNA) molecule.

The GPC3-specific CAR of the present disclosure or the GPC3-specific humanized antibody-derived CAR of the present disclosure may be prepared by any method known in the art. After encoding the polypeptide sequence of the GPC3-specific CAR of the present disclosure or the GPC3-specific humanized antibody-derived CAR of the present disclosure, if desired, the polypeptide sequence is cloned into a host cell and used to form nucleic acids to be expressed and assayed.

The nucleic acid encoding the GPC3-specific CAR of the present disclosure or the GPC3-specific humanized antibody-derived CAR of the present disclosure may be inserted into an expression vector for protein expression.

In an aspect, the present disclosure provides a vector including a polynucleotide including a nucleic acid sequence encoding a GPC3-specific CAR according to the present disclosure or a GPC3-specific humanized antibody-derived CAR according to the present disclosure.

In an aspect, the present disclosure provides a method for producing a GPC3-specific CAR or a GPC3-specific humanized antibody-derived CAR, including infecting a host cell with a vector including a polynucleotide including a nucleic acid sequence encoding a GPC3-specific CAR according to the present disclosure or a GPC3-specific humanized antibody-derived CAR according to the present disclosure.

The vector of the present disclosure and the method for producing the GPC3-specific CAR or the GPC3-specific humanized antibody-derived CAR using the same may be equally applied with the description disclosed in the antibody or the fragment having immunological activity thereof, or the humanized antibody or the antigen binding fragment thereof of the present disclosure.

In an aspect, the present disclosure relates to a chimeric antigen receptor expressing cell transformed with the GPC3-specific CAR of the present disclosure.

In an aspect, the present disclosure relates to a chimeric antigen receptor expressing cell transformed with the GPC3-specific humanized antibody-derived CAR of the present disclosure.

In an exemplary embodiment, the chimeric antigen receptor expressing cell may be a chimeric antigen receptor expressing macrophage (CAR-macrophage), a chimeric antigen receptor expressing T (CAR-T) cell, a chimeric antigen receptor expressing-gamma-delta T (CAR-Gamma-delta T) cell, or a chimeric antigen receptor natural killer (CAR-NK) cell.

As used herein, the term "chimeric antigen receptor expressing T (CAR-T) cell" means a T cell expressing a CAR. The chimeric antigen receptor expressing T (CAR-T) cell i) recognizes a cancer antigen in a manner independent of a human leukocyte antigen (HLA) to have the advantage of being able to treat cancers that avoid the action of anticancer drugs by reducing HLA expression on the cell surface, ii) is independent of an HLA type to be used for treatment regardless of the patient's HLA type, and iii) has the advantage of being able to produce a large number of cancer-specific T cells in a short period of time to exhibit excellent anticancer effects.

The T cell includes CD4⁺ T cells (helper T cells, TH cells), CD8⁺ T cells (cytotoxic T cells, CTLs), memory T cells, regulatory T cells (Treg cells), natural killer T cells, etc., and the T cells introduced with the CAR in the present disclosure are preferably CD8⁺ T cells, but are not limited thereto.

In an aspect, the present disclosure provides a pharmaceutical composition for preventing or treating cancer including: a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

The pharmaceutical composition of the present disclosure may be equally applied with the description disclosed in the antibody or the fragment having immunological activity thereof, or the humanized antibody or the antigen binding fragment thereof of the present disclosure.

In an aspect, the present disclosure provides a cell therapeutic agent for preventing or treating cancer including a chimeric antigen receptor expressing cell transformed with a GPC3-specific CAR according to the present disclosure or a chimeric antigen receptor expressing cell transformed with a GPC3-specific humanized antibody-derived CAR according to the present disclosure.

As used herein, the term "a cell therapeutic agent" refers to a medicine (under US FDA regulations) used for the purposes of treatment, diagnosis, and prevention, which consists of cells and tissues prepared through isolation, culture, and special manipulation from a subject, and means a medicine used for treatment, diagnosis and prevention through a series of actions, such as *ex vivo* proliferating and selecting living autologous, allogeneic, or xenogeneic cells to restore the functions of cells or tissues, changing the biological characteristics of cells by other methods, or the like.

In an aspect, the present disclosure provides an anticancer adjuvant including a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

In an exemplary embodiment, the anticancer adjuvant of the present disclosure may be co-administered concurrently, separately, or sequentially with a cancer immunotherapy.

In an exemplary embodiment, the anticancer adjuvant may include reducing refractoriness to cancer immunotherapy.

In an exemplary embodiment, the cancer immunotherapy may be immune checkpoint inhibitors, an immunosuppressant control drug, a cancer vaccine, an immunoadjuvant, an immune cell for cancer treatment, an immune cell activation cofactor, an antibody for cancer treatment, or cytokines required to maintain the activity of an immune cell for cancer treatment.

In an exemplary embodiment, the immune checkpoint inhibitor may be an inhibitor of CTLA-4, PD-1, PD-L1, PD-L2, LAG-3, BTLA, B7H3, B7H4, TIM3, KIR, TIGIT, CD47, VISTA or A2aR.

In an aspect, the present disclosure provides a composition for diagnosing cancer including a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

In an aspect, the present disclosure provides a kit for diagnosing cancer including a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

In an exemplary embodiment, the composition for diagnosing cancer and the kit for diagnosing cancer of the present disclosure may diagnose cancer by detecting GPC3, which is a cancer antigen.

The composition for detecting cancer and the kit for detecting cancer of the present disclosure may be equally applied with the description disclosed in the antibody or the fragment having immunological activity thereof, or the humanized antibody or the antigen binding fragment thereof of the present disclosure.

In an aspect, the present disclosure provides a method for providing information for the diagnosis of cancer including: treating a sample with a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure, which is labeled with a fluorescent substance; and measuring the expression of the GPC3-specific CAR, the GPC3-specific humanized antibody-derived CAR, the chimeric antigen receptor expressing cell transformed with the GPC3-specific CAR, or the chimeric antigen receptor expressing cell transformed with the GPC3-specific humanized antibody-derived CAR.

In an exemplary embodiment, when the expression of the GPC3-specific CAR, the GPC3-specific humanized antibody-derived CAR, the chimeric antigen receptor expressing cell transformed with the GPC3-specific CAR, or the chimeric antigen receptor expressing cell transformed with the GPC3-specific humanized antibody-derived CAR is measured, it may be diagnosed as having developed cancer.

The method for providing information of the present disclosure may be equally applied with the description disclosed in the antibody or the fragment having immunological activity thereof, or the humanized antibody or the antigen binding fragment thereof of the present disclosure.

In an aspect, the present disclosure provides a method for detecting cancer antigens including: administering, to a subject, an isolated antibody or a fragment having immunological activity thereof according to the present disclosure, a humanized antibody or an antigen binding fragment thereof according to the present disclosure, a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

In an exemplary embodiment, the cancer antigen may be GPC3.

In addition, in an aspect, the present disclosure provides a method for preventing or treating cancer including: administering, to a subject, an antibody-drug conjugate according to the present disclosure, a GPC3-specific CAR according to the present disclosure, a GPC3-specific humanized antibody-derived CAR according to the present disclosure, a chimeric antigen receptor expressing cell transformed with a GPC3-specific CAR according to the present disclosure, a chimeric antigen receptor expressing cell transformed with a GPC3-specific humanized antibody-derived CAR according to the present disclosure, a cell therapeutic agent including a chimeric antigen receptor expressing cell transformed with a GPC3-specific CAR according to the present disclosure, or a cell therapeutic agent including a chimeric antigen receptor expressing cell transformed with a GPC3-specific humanized antibody-derived CAR according to the present disclosure.

In an exemplary embodiment, the cancer may be refractory to an immune checkpoint inhibitor.

In an exemplary embodiment, the cancer may be at least any one selected from the group consisting of brain tumor, melanoma, myeloma, non-small cell lung cancer, oral cancer, liver cancer, stomach cancer, colon cancer, breast cancer, triple negative breast cancer (TNBC), lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph node cancer, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or ureteral cancer, renal cell carcinoma, renal pelvic carcinoma, central nervous system tumor, primary central nervous system lymphoma, spinal cord tumor, brainstem glioma and pituitary adenoma, but is not limited thereto.

In an aspect, the present disclosure provides a method for enhancing an anticancer treatment effect in a subject including: administering, to a subject, a GPC3-specific CAR according to the present disclosure, a GPC3-specific humanized antibody-derived CAR according to the present disclosure, a chimeric antigen receptor expressing cell transformed with a GPC3-specific CAR according to the present disclosure, or a chimeric antigen receptor expressing cell transformed with a GPC3-specific humanized antibody-derived CAR according to the present disclosure.

In an exemplary embodiment, the enhancing of the anticancer treatment effect in the subject may include reducing refractoriness to cancer immunotherapy.

The subject is preferably mammals, including humans, and may include patients who need to be checked for the presence of cancer antigens or patients requiring treatment for cancer, including all patients currently undergoing treatment, patients who have received treatment, and patients who need to receive treatment, and may also include patients who have undergone surgical procedures for the treatment of cancer.

Hereinafter, the present disclosure will be described in more detail through the following Examples. However, the following Examples are only intended to embody the contents of the present disclosure, and the present disclosure is not limited thereto.

### Example 1. Preparation of Glypican-3 (GPC3)-specific antibody

### 1-1. Preparation of antibody

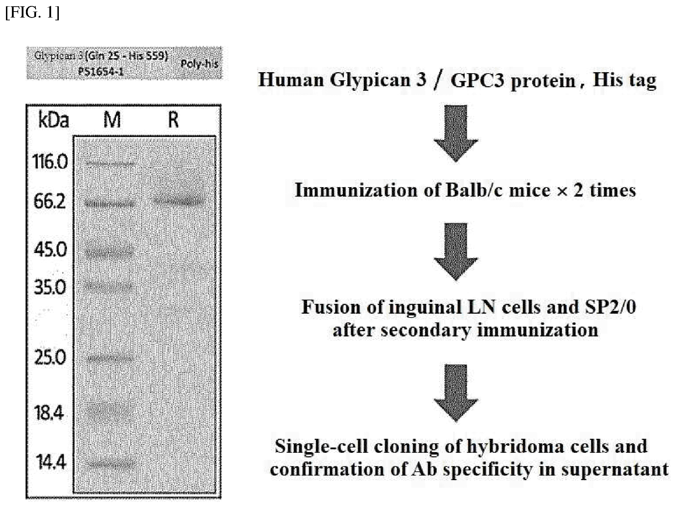

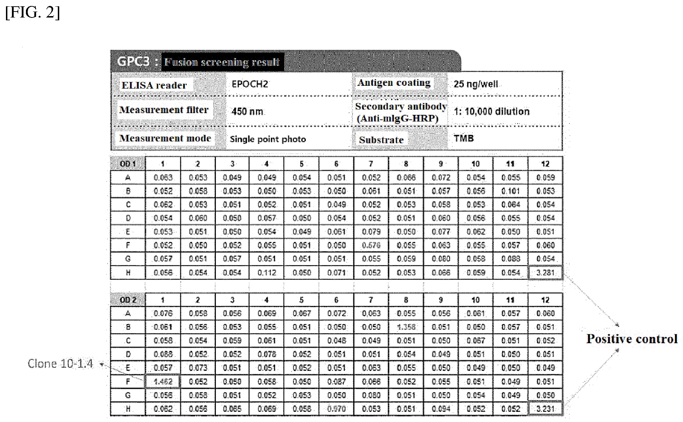

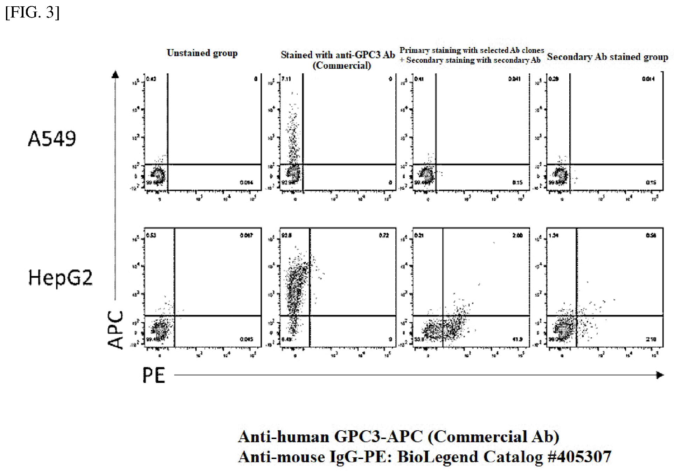

Balb/c wild-type mice were immune-induced with a GPC3 protein twice, and after the second immune-induction, immune cells in the lymph nodes were isolated and fused with an SP2/0 cell line to prepare hybridoma cells. The prepared hybridoma cells were cultured as single-cell clones, and to confirm the specificity to GPC3 of each clone, the supernatant of each clone was analyzed using ELISA to derive 10-1.4, a hybridoma clone specific to GPC3 (FIGS. 1 and 2). Subsequently, HepG2 cell lines overexpressing GPC3 and A549 cells (negative control) were divided into the following groups, stained with each antibody, and then flow cytometry was performed to confirm the GPC3-specific binding of the 10-1.4 antibody clone (FIG. 3): an unstained group (Unstained); a positive control group stained with anti-human GPC3:APC antibody (Anti-GPC3 Ab); a group secondary stained with anti-mouse IgG:PE antibody after primary stained with a culture supernatant of the selected hybridoma clone 10-1.4 (Isolated Ab clone + 2' Ab); and a group secondary stained with anti-mouse IgG:PE antibody without primary staining (2' Ab only).

Through this, the GPC3-specific hybridoma antibody clone 10-1.4 was derived.

### 1-2. Antibody sequencing analysis

RT-PCR and cDNA sequencing were performed on the GPC3-specific hybridoma antibody clone 10-1.4 derived in Example 1-1 above to analyze sequences of a VH region (amino acid sequence: SEQ NO ID: 7; and nucleotide sequence: SEQ NO ID: 9) and a VL region (amino acid sequence: SEQ NO ID: 8; and nucleotide sequence: SEQ NO ID: 10) of a GPC3-specific antibody and confirm respective signal sequences of FR1-CDR1-FR2-CDR2-FR3-CDR3-FR4 of VH and VL (FIG. 4).

### 1-3. Preparation of scFv

The hybridoma antibody clone 10-1.4 sequenced above was expressed with an scFv format protein of FLAG tag-VL-(linker)-VH, and then HepG2 cell lines overexpressing GPC3 were divided into the following groups, stained with each antibody, and flow cytometry was performed to confirm the binding ability to GPC3: an unstained group; a positive control group stained with anti-human GPC3:APC antibody; and a group secondary stained with anti-FLAG tag:APC antibody after primary staining with FLAG tag-scFv format protein of selected hybridoma clone 10-1.4.

As a result, it was found that scFv of the hybridoma clone 10-1.4 specifically bound to GPC3 (FIG. 5).

### Example 2. Preparation of GPC3 CAR-T and confirmation of antigen specificity

To prepare GPC3 CAR-T, a chimeric antigen receptor (CAR) including GPC3 scFv as an antigen binding domain was transduced into T cells (FIG. 6). Specifically, a vector was constructed in the form of a CAR by linking a FLAG-tag sequence (DYKDDDDK) to an N-terminal portion of an scFv sequence of the antibody clone 10-1.4 prepared in Example 1 above and linking a chimeric antigen receptor (CAR) sequence (CD28-CD3z) to a C-terminal portion, and a lentivirus was constructed using the vector. In addition, CD4/CD8-positive T cells were isolated from peripheral blood derived from a normal donor, and the T cells were stimulated by contacting the cells with microbeads coated with anti-human CD2/3/28 antibody. One day after T cell stimulation, the lentivirus including the CAR prepared above was brought into contact with the T cells to transduce the gene into the T cells. Thereafter, CAR-T was proliferated through a process of checking the cell count at intervals of 2 to 3 days and re-culturing the cells, and on day 11, the CAR-T was harvested and then stained with anti-human CD8:FITC and anti-FLAG tag:APC to confirm a stable CAR expression pattern on the cell surface by flow cytometry. In addition, in order to confirm whether the GPC3 CAR expressed on T cells recognized and bound to a GPC3 antigen, the GPC3 CAR-T of the present disclosure prepared by the method of FIG. 5 was stained with soluble GPC3:APC protein and anti-human CD8:FITC, and then flow cytometry was performed. As a negative control group, CAR-T having scFv of an FMC63 antibody clone that recognized CD19 was stained and compared.

As a result, it was found that the GPC3 CAR-T of the present disclosure stably expressed CAR on the cell surface (FIG. 7A), and that the GPC3 CAR expressed on the T cells recognized and bound to the GPC3 antigen (FIG. 7B).

### Example 3. Confirmation of GPC3-specific anticancer immune response of GPC3 CAR-T

To confirm a GPC3-specific anticancer immune response of GPC3 CAR-T, CD19 CAR-T (negative control) and GPC3 CA-T were co-cultured with an HepG2 cell line to determine whether the CAR-T exhibited an antigen-specific immune response. Specifically, each CAR-T was added to a container in which HepG2 cells were cultured, and a protein transporter inhibitor cocktail (eBioscience) was added to the culture medium to inhibit exocytosis of cytokines. After 6 hours of co-culture, the expression of cytokines was confirmed by flow cytometry using extracellular anti-CD8:FITC staining and intracellular cytokine staining (anti-human IL2:BV421, anti-human TNF:PerCP-Cy5.5 and anti-human IFN-γ:PE-Cy7).

As a result, it was confirmed that the GPC3 CAR-T of the present disclosure reacted with GPC3 of the HepG2 cell line to increase the expression of cytokines IL-2, TNF, and IFN-γ (FIG. 8).

### Example 4. Preparation of GPC3 CAR-T having CD28 or 4-1BB as co-stimulatory domain and confirmation of antigen specificity

GPC3 BBz and GPC3 28z CAR-Ts having CD28 or 4-1BB as a co-stimulatory domain were prepared in the same manner as in Example 2 above. Specifically, a vector was constructed in the form of a CAR by linking a FLAG-tag sequence (DYKDDDDK) to an N-terminal portion of the scFv sequence of the antibody clone 10-1.4 prepared in Example 1 above and linking a CAR sequence (4-1BB-CD3z or CD28-CD3z) to a C-terminal portion, and a lentivirus was prepared using the vector. The lentivirus was introduced into T cells to construct GPC3 CAR-T having CD28 or 4-1BB as a co-stimulatory domain, and flow cytometry was performed by staining with anti-human CD8:FITC and anti-FLAG tag:APC, and the results were shown in FIG. 9. In FIG. 9 above, CD19 BBz refers to a CAR-T having scFv of an FMC63 antibody clone used as a control and a 4-1BB co-stimulatory domain, CD19 28z refers to a CAR-T having scFv of an FMC63 antibody clone used as a control and a CD28 co-stimulatory domain, GPC3 BBz refers to a CAR-T having an scFv sequence of the clone 10-1.4 and the 4-1BB co-stimulatory domain, and GPC3 28z refers to a CAR-T having an scFv sequence of the clone 10-1.4 and the CD28 co-stimulatory domain.

As a result, as shown in FIG. 9, it was confirmed that GPC3 BBz and GPC3 28z CAR-T having CD28 or 4-1BB as a co-stimulatory domain according to the present disclosure were each stably expressed on the cell surface.

In addition, whether the GPC3 BBz and GPC3 28z CAR-T exhibited a specific anticancer immune response against GPC3 was confirmed in the same manner as in Example 3 and was shown in FIG. 10. In FIG. 10, CD19 BBz refers to a CAR-T having scFv of an FMC63 antibody clone used as a control and a 4-1BB co-stimulatory domain, CD19 28z refers to a CAR-T having scFv of an FMC63 antibody clone used as a control and a CD28 co-stimulatory domain, GPC3 BBz refers to a CAR-T having an scFv sequence of the clone 10-1.4 and the 4-1BB co-stimulatory domain, and GPC3 28z refers to a CAR-T having an scFv sequence of the clone 10-1.4 and the CD28 co-stimulatory domain.

As a result, as shown in FIG. 10, it was confirmed that GPC3 BBz and GPC3 28z CAR-T having CD28 or 4-1BB as a co-stimulatory domain according to the present disclosure exhibited GPC3-specific anticancer immune responses.

### Example 5. Confirmation of in vivo anticancer effect of GPC3 CAR-T having CD28 or 4-1BB as co-stimulatory domain

To confirm an *in vivo* effect of the GPC3 CAR-T according to the present disclosure, cancer growth was induced by first inoculating an HepG2 cell line overexpressing a human CD19 protein and Firefly luciferase into severe immunodeficient mice, NOG (NOD.Cg-Prkdcscid II2rgtm1Sug/Jic).

In addition, CD19 CAR-T (CAR having 4-1BB or CD28 as a co-stimulatory domain) and GPC3 CAR-T (CAR having CD28 as a co-stimulatory domain) were constructed in the same manner as in Example 2 above, injected into mice inoculated with the HepG2-hCD19-luc2, and cancer inhibitory ability was confirmed by measuring luciferase activity.

As a result, as shown in FIG. 11, it was confirmed that cancer growth was inhibited in mice injected with GPC3 CAR-T. In particular, it was confirmed that the GPC3 CAR-T showed equivalent or superior anticancer efficacy compared to mice administered with previously approved CD19 CAR-T.

### Example 6. Preparation of humanized antibody using GPC3-specific hybridoma antibody and confirmation of antigen specificity

A humanized antibody was prepared through a humanization process using an antibody sequence of GPC3-specific hybridoma clone 10-1.4 according to the present disclosure. Specifically, as shown in FIG. 12, a total of 16 types of humanized antibody sequences, designated v1 to v16, were designed by grafting CDR1 to CDR3 sequences of VH and VL regions of clone 10-1.4 into four types of human V_{H} frames and four types of human V_{L} frames, and then expressing the sequences in combination. When each antibody sequence was expressed in HEK293T cells, it was confirmed that 10 types of humanized antibodies, excluding v9 to v14, were stably secreted into the cell culture medium in an antibody form (bottom of FIG. 12 (yield obtained by purifying 80 mL of supernatant by affinity chromatography)).

In addition, HepG2 and Huh-7 cell lines overexpressing GPC3, Nalm6 as a negative control, and Nalm6-GPC3 cells (overexpressing GPC3) as a positive control were stained with the 10 types of humanized antibodies, respectively, and then secondary stained with anti-hIgG-Fc:PE, and flow cytometry was performed as shown in FIG. 13. In FIG. 13 above, 2nd only refers to a group stained only with the secondary antibody anti-hIgG-Fc:PE, Mouse refers to a group stained with a chimeric antibody produced by linking the VH and VL sequences of the original mouse antibody, clone 10-1.4 to the human CH and CL sequences, and v1 to v16 refer to groups stained with each humanized antibody. As a result, it was confirmed that all of the humanized antibodies according to the present disclosure exhibited GPC3 antigen specificity.

### Example 7. Preparation of CAR-T using humanized antibody and confirmation of antigen specificity

CAR-T was prepared using humanized antibodies v1, v3, v6, and v8 in the same manner as in Example 2 above. More specifically, a vector was constructed in the form of a second-generation CAR by linking a FLAG-tag sequence (DYKDDDDK (SEQ ID NO: 34)) to the N-terminal portion of the scFv sequences of the humanized antibodies v1, v3, v6, and v8 and linking a CAR sequence (CD28-CD3z) to the C-terminal portion. GPC3 CAR-T was prepared by constructing a lentivirus with the corresponding vector and introducing the lentivirus into T cells. The GPC3 CAR-T was stained with anti-human CD8:FITC and anti-FLAG tag:APC and simultaneously interacted with a soluble GPC3:APC protein to confirm by flow cytometry whether the GPC3 CAR expressed on T cells recognized and bound to the GPC3 antigen. At this time, samples stained with serially diluted concentrations of soluble GPC3:APC protein were simultaneously analyzed. As a result, as shown in FIG. 14, it was confirmed that the GPC3 antibody according to the present disclosure was stably expressed in T cells in the form of a humanized CAR sequence. In addition, it was confirmed that V1 and V3 showed excellent binding affinity to the GPC3 antigen, while V6 and V8 showed somewhat lower affinity. In FIG. 14, Original was an experimental group for CAR-T having a mouse form (pre-humanization) scFv sequence of clone 10-1.4 used as a positive control.

Novel antibodies specifically binding to GPC3 prepared in the present disclosure and amino acid sequences and nucleotide sequences of CAR-T using the same were as shown in Table 1 below.

**[Table 1]**

| **Name** | **Sequence** | **SEQ ID NO.** |
|---|---|---|
| CDRH1 | TYAMN | 1 |
| CDRH2 | RIRSKSNNYA TYYADSVKD | 2 |
| CDRH3 | LGYRYDGYAM DY | 3 |
| CDRL1 | SASSSVSYMY | 4 |
| CDRL2 | DTSKLAS | 5 |
| CDRL3 | QQWSSNPPT | 6 |
| GPC3 antibody VH domain | MLLGLKWVFF VVFYQGVHCE VQLVESGGGL VQPKGSLKLS CAASGFTFNT YAMNWVRQAP GKGLEWVARI RSKSNNYATY YADSVKDRFT ISRDDSQSML YLQMNNLKTE DTAMYYCVRL GYRYDGYAMD YWGQGTSVTV SS | 7 |
| GPC3 antibody VL domain | MDFQVQIFSF LLISASVIIS RGQIVLTQSP AIMSASPGEK VTMTCSASSS VSYMYWYQQK SGTSPKRWIY DTSKLASGVP ARFSGSGSGT | 8 |
| | SYSLTISSME AEDAATYYCQ QWSSNPPTFG GGTKLEIK | |
| GPC3 antibody VH domain sequence | atgctgttgg ggctgaagtg ggttttcttt gttgtttttt atcaaggtgt gcattgtgag gtgcagcttg ttgagtctgg tggaggattg gtgcagccta aagggtcatt gaaactctca tgtgcagcct ctggattcac cttcaatacc tacgccatga actgggtccg ccaggctcca ggaaagggtt tggaatgggt tgctcgcata agaagtaaaa gtaataatta tgcaacatat tatgccgatt cagtgaaaga caggttcacc atctccagag atgattcaca aagcatgctc tatctgcaaa tgaacaactt gaaaactgag gacacagcca tgtattactg tgtgagactt ggctataggt acgacggcta tgctatggac tactggggtc aaggaacctc agtcaccgtc tcctca | 9 |
| GPC3 antibody VL domain sequence | atggattttc aagtgcagat tttcagcttc ctgctaatca gtgcctcagt cataatatcc agaggacaaa ttgttctcac ccagtctcca gcaatcatgt ctgcatctcc aggggagaag gtcaccatga cctgcagtgc cagctcaagt gtaagttata tgtattggta ccagcagaag tcaggcacct cccccaaaag atggatttat gacacatcca aactggcttc tggagtccct gctcgcttca gtggcagtgg gtctgggacc tcttactctc tcacaatcag cagcatggag gctgaagatg ctgccactta ttactgccag cagtggagta gtaacccacc cacgttcgga ggggggacca agctggaaat aaaa | 10 |
| Humanized VH1 domain | EVQLVESGGGLVQPGGSLRLSCAASGFTFNTYAMNWV RQAPGKGLEWVARIRSKSNNYATYYADSVKGRFTISRD DSQSMLYLQMNSLKTEDTAVYYCVRLGYRYDGYAMD YWGQGTTVTVSS | 11 |
| Humanized VH2 domain | EVQLVESGGGLVQPGGSLRLSCAASGFTFNTYAMNWV RQAPGKGLEWVARIRSKSNNYATYYADSVKGRFTISRD DSKNSLYLQMNSLKTEDTAVYYCVRLGYRYDGYAMD YWGQGTTVTVSS | 12 |
| Humanized VH3 domain | QVQLQESGPGLVKPSETLSLTCTASGFTFNTYAMNWVR QPPGKGLEWVARIRSKSNNYATYYADSLKSRFTISRDD SQSMLSLKLSSVTAADTAVYYCVRLGYRYDGYAMDY WGQGTTVTVSS | 13 |
| Humanized VH4 domain | QVQLQESGPGLVKPSETLSLTCTASGFTFNTYAMNWIR QPPGKGLEWIARIRSKSNNYATYYADSLKSRVTISRDDS KNQLSLKLSSVTAADTAVYYCVRLGYRYDGYAMDYW GQGTTVTVSS | 14 |
| Humanized VL1 domain | QIVLTQSPATLSLSPGERATLSCRASSSVSYMYWYQQK PGQAPRRWIYDTSKLASGVPARFSGSGSGTSYSLTISSL EPEDFATYYCQQWSSNPPTFGGGTKLEIK | 15 |
| Humanized VL2 domain | EIVLTQSPATLSLSPGERATLSCRASSSVSYMYWYQQKP GQAPRRLIYDTSKRATGIPARFSGSGSGTDYTLTISSLEP EDFAVYYCQQWSSNPPTFGGGTKLEIK | 16 |
| Humanized VL3 domain | QIQLTQSPSSLSASVGDRVTITCRASSSVSYMYWYQQK PGKAPKRWIYDTSKLASGVPSRFSGSGSGTSYSLTISSL QPEDFATYYCQQWSSNPPTFGGGTKLEIK | 17 |
| Humanized | DIQLTQSPSSLSASVGDRVTITCRASSSVSYMYWYQQK | 18 |
| VL4 domain | PGKAPKRLIYDTSKLASGVPSRFSGSGSGTDYTLTISSL QPEDFATYYCQQWSSNPPTFGGGTKLEIK | |
| CAR-T_GPC antibody VL | QIVLTQSPAIMSASPGEKVTMTCSASSSVSYMYWYQQ KSGTSPKRWIYDTSKLASGVPARFSGSGSGTSYSLTISS MEAEDAATYYCQQWSSNPPTFGGGTKLEIK | 19 |
| CAR-T_GPC antibody VH | EVQLVESGGGLVQPKGSLKLSCAASGFTFNTYAMNWV RQAPGKGLEWVARIRSKSNNYATYYADSVKDRFTISRD DSQSMLYLQMNNLKTEDTAMYYCVRLGYRYDGYAM DYWGQGTSVTVSS | 20 |
| GS linker | GGGGSGGGGSGGGGS | 21 |
| GPC antibody CAR | QIVLTQSPAIMSASPGEKVTMTCSASSSVSYMYWYQQ KSGTSPKRWIYDTSKLASGVPARFSGSGSGTSYSLTISS MEAEDAATYYCQQWSSNPPTFGGGTKLEIKGGGGSGG GGSGGGGSEVQLVESGGGLVQPKGSLKLSCAASGFTF NTYAMNWVRQAPGKGLEWVARIRSKSNNYATYYADS VKDRFTISRDDSQSMLYLQMNNLKTEDTAMYYCVRL GYRYDGYAMDYWGQGTSVTVSS | 22 |
| v1 CAR scFv_VH1VL 1 | QIVLTQSPATLSLSPGERATLSCRASSSVSYMYWYQQK PGQAPRRWIYDTSKLASGVPARFSGSGSGTSYSLTISSL EPEDFATYYCQQWSSNPPTFGGGTKLEIKGGGGSGGG GSGGGGSEVQLVESGGGLVQPGGSLRLSCAASGFTFNT YAMNWVRQAPGKGLEWVARIRSKSNNYATYYADSVK GRFTISRDDSQSMLYLQMNSLKTEDTAVYYCVRLGYR YDGYAMDYWGQGTTVTVSS | 23 |
| v3 CAR scFv_VH1VL 3 | QIQLTQSPSSLSASVGDRVTITCRASSSVSYMYWYQQK PGKAPKRWIYDTSKLASGVPSRFSGSGSGTSYSLTISSL QPEDFATYYCQQWSSNPPTFGGGTKLEIKGGGGSGGG GSGGGGSEVQLVESGGGLVQPGGSLRLSCAASGFTFNT YAMNWVRQAPGKGLEWVARIRSKSNNYATYYADSVK GRFTISRDDSQSMLYLQMNSLKTEDTAVYYCVRLGYR YDGYAMDYWGQGTTVTVSS | 24 |
| v6 CAR scFv_VH2VL 2 | EIVLTQSPATLSLSPGERATLSCRASSSVSYMYWYQQKP GQAPRRLIYDTSKRATGIPARFSGSGSGTDYTLTISSLEP EDFAVYYCQQWSSNPPTFGGGTKLEIKGGGGSGGGGS GGGGSEVQLVESGGGLVQPGGSLRLSCAASGFTFNTYA MNWVRQAPGKGLEWVARIRSKSNNYATYYADSVKGR FTISRDDSKNSLYLQMNSLKTEDTAVYYCVRLGYRYD GYAMDYWGQGTTVTVSS | 25 |
| v8 CAR scFv_VH2VL 4 | DIQLTQSPSSLSASVGDRVTITCRASSSVSYMYWYQQK PGKAPKRLIYDTSKLASGVPSRFSGSGSGTDYTLTISSL QPEDFATYYCQQWSSNPPTFGGGTKLEIKGGGGSGGG GSGGGGSEVQLVESGGGLVQPGGSLRLSCAASGFTFNT YAMNWVRQAPGKGLEWVARIRSKSNNYATYYADSVK GRFTISRDDSKNSLYLQMNSLKTEDTAVYYCVRLGYR YDGYAMDYWGQGTTVTVSS | 26 |
| GPC3 CAR (10-1.4 original mouse CD8a signal-FLAG tag-VL-GS linker-VH-CD28-CD3z) | MALPVTALLLPLALLLHAARPDYKDDDDKQIVLTQSP AIMSASPGEKVTMTCSASSSVSYMYWYQQKSGTSPKR WIYDTSKLASGVPARFSGSGSGTSYSLTISSMEAEDAAT YYCQQWSSNPPTFGGGTKLEIKGGGGSGGGGSGGGGS EVQLVESGGGLVQPKGSLKLSCAASGFTFNTYAMNWV RQAPGKGLEWVARIRSKSNNYATYYADSVKDRFTISRD DSQSMLYLQMNNLKTEDTAMYYCVRLGYRYDGYAM DYWGQGTSVTVSSIEVMYPPPYLDNEKSNGTIIHVKGK HLCPSPLFPGPSKPFWVLVVVGGVLACYSLLVTVAFIIF WVRSKRSRLLHSDYMNMTPRRPGPTRKHYQPYAPPR DFAAYRSRVKFSRSADAPAYQQGQNQLYNELNLGRRE EYDVLDKRRGRDPEMGGKPRRKNPQEGLYNELQKDK MAEAYSEIGMKGERRRGKGHDGLYQGLSTATKDTYD ALHMQALPPR | 27 |
| GPC3 CAR (10-1.4 original mouse CD8a signal-FLAG tag-VL-GS linker-VH-CD28-CD3z) | MALPVTALLLPLALLLHAARPDYKDDDDKQIVLTQSP AIMSASPGEKVTMTCSASSSVSYMYWYQQKSGTSPKR WIYDTSKLASGVPARFSGSGSGTSYSLTISSMEAEDAAT YYCQQWSSNPPTFGGGTKLEIKGGGGSGGGGSGGGGS EVQLVESGGGLVQPKGSLKLSCAASGFTFNTYAMNWV RQAPGKGLEWVARIRSKSNNYATYYADSVKDRFTISRD DSQSMLYLQMNNLKTEDTAMYYCVRLGYRYDGYAM DYWGQGTSVTVSSIEVMYPPPYLDNEKSNGTIIHVKGK HLCPSPLFPGPSKPFWVLVVVGGVLACYSLLVTVAFIIF WVRSKRSRLLHSDYMNMTPRRPGPTRKHYQPYAPPR DFAAYRSRVKFSRSADAPAYQQGQNQLYNELNLGRRE EYDVLDKRRGRDPEMGGKPRRKNPQEGLYNELQKDK MAEAYSEIGMKGERRRGKGHDGLYQGLSTATKDTYD ALHMQALPPR | 28 |
| GPC3 CAR (10-1.4 original mouse CD8a signal-FLAG tag-VL-GS linker-VH-CD8 transmembran e-4-1BB-CD3z) | MALPVTALLLPLALLLHAARPDYKDDDDKQIVLTQSP AIMSASPGEKVTMTCSASSSVSYMYWYQQKSGTSPKR WIYDTSKLASGVPARFSGSGSGTSYSLTISSMEAEDAAT YYCQQWSSNPPTFGGGTKLEIKGGGGSGGGGSGGGGS EVQLVESGGGLVQPKGSLKLSCAASGFTFNTYAMNWV RQAPGKGLEWVARIRSKSNNYATYYADSVKDRFTISRD DSQSMLYLQMNNLKTEDTAMYYCVRLGYRYDGYAM DYWGQGTSVTVSSTTTPAPRPPTPAPTIASQPLSLRPEA CRPAAGGAVHTRGLDFACDIYIWAPLAGTCGVLLLSLV ITLYCKRGRKKLLYIFKQPFMRPVQTTQEEDGCSCRFPE EEEGGCELRVKFSRSADAPAYQQGQNQLYNELNLGRR EEYDVLDKRRGRDPEMGGKPRRKNPQEGLYNELQKD KMAEAYSEIGMKGERRRGKGHDGLYQGLSTATKDTY DALHMQALPPR | 29 |
| Humanized v1 GPC3 CAR (CD8a signal-FLAG tag-VL-GS linker-VH-CD28-CD3z) | MALPVTALLLPLALLLHAARPDYKDDDDKQIVLTQSPA TLSLSPGERATLSCRASSSVSYMYWYQQKPGQAPRRW IYDTSKLASGVPARFSGSGSGTSYSLTISSLEPEDFATYY | 30 |
| | CQQWSSNPPTFGGGTKLEIKGGGGSGGGGSGGGGSEV QLVESGGGLVQPGGSLRLSCAASGFTFNTYAMNWVRQ APGKGLEWVARIRSKSNNYATYYADSVKGRFTISRDDS QSMLYLQMNSLKTEDTAVYYCVRLGYRYDGYAMDY WGQGTTVTVSSIEVMYPPPYLDNEKSNGTIIHVKGKHL CPSPLFPGPSKPFWVLVVVGGVLACYSLLVTVAFIIFWV RSKRSRLLHSDYMNMTPRRPGPTRKHYQPYAPPRDFA AYRSRVKFSRSADAPAYQQGQNQLYNELNLGRREEYD VLDKRRGRDPEMGGKPRRKNPQEGLYNELQKDKMAE AYSEIGMKGERRRGKGHDGLYQGLSTATKDTYDALH MQALPPR | |
| Humanized v3 GPC3 CAR (CD8a signal-FLAG tag-VL-GS linker-VH-CD28-CD3z) | MALPVTALLLPLALLLHAARPDYKDDDDKQIQLTQSPS SLSASVGDRVTITCRASSSVSYMYWYQQKPGKAPKRW IYDTSKLASGVPSRFSGSGSGTSYSLTISSLQPEDFATYY CQQWSSNPPTFGGGTKLEIKGGGGSGGGGSGGGGSEV QLVESGGGLVQPGGSLRLSCAASGFTFNTYAMNWVRQ APGKGLEWVARIRSKSNNYATYYADSVKGRFTISRDDS QSMLYLQMNSLKTEDTAVYYCVRLGYRYDGYAMDY WGQGTTVTVSSIEVMYPPPYLDNEKSNGTIIHVKGKHL CPSPLFPGPSKPFWVLVVVGGVLACYSLLVTVAFIIFWV RSKRSRLLHSDYMNMTPRRPGPTRKHYQPYAPPRDFA AYRSRVKFSRSADAPAYQQGQNQLYNELNLGRREEYD VLDKRRGRDPEMGGKPRRKNPQEGLYNELQKDKMAE AYSEIGMKGERRRGKGHDGLYQGLSTATKDTYDALH MQALPPR | 31 |
| Humanized v6 GPC3 CAR (CD8a signal-FLAG tag-VL-GS linker-VH-CD28-CD3z) | MALPVTALLLPLALLLHAARPDYKDDDDKEIVLTQSPA TLSLSPGERATLSCRASSSVSYMYWYQQKPGQAPRRLI YDTSKRATGIPARFSGSGSGTDYTLTISSLEPEDFAVYYC QQWSSNPPTFGGGTKLEIKGGGGSGGGGSGGGGSEVQ LVESGGGLVQPGGSLRLSCAASGFTFNTYAMNWVRQA PGKGLEWVARIRSKSNNYATYYADSVKGRFTISRDDSK NSLYLQMNSLKTEDTAVYYCVRLGYRYDGYAMDYWG QGTTVTVSSIEVMYPPPYLDNEKSNGTIIHVKGKHLCP SPLFPGPSKPFWVLVVVGGVLACYSLLVTVAFIIFWVRS KRSRLLHSDYMNMTPRRPGPTRKHYQPYAPPRDFAAY RSRVKFSRSADAPAYQQGQNQLYNELNLGRREEYDVL DKRRGRDPEMGGKPRRKNPQEGLYNELQKDKMAEAY SEIGMKGERRRGKGHDGLYQGLSTATKDTYDALHMQ ALPPR | 32 |
| Humanized v8 GPC3 CAR (CD8a signal-FLAG tag-VL-GS linker-VH-CD28-CD3z) | MALPVTALLLPLALLLHAARPDYKDDDDKDIQLTQSPS SLSASVGDRVTITCRASSSVSYMYWYQQKPGKAPKRLI YDTSKLASGVPSRFSGSGSGTDYTLTISSLQPEDFATYY CQQWSSNPPTFGGGTKLEIKGGGGSGGGGSGGGGSEV QLVESGGGLVQPGGSLRLSCAASGFTFNTYAMNWVRQ APGKGLEWVARIRSKSNNYATYYADSVKGRFTISRDDS | 33 |
| | KNSLYLQMNSLKTEDTAVYYCVRLGYRYDGYAMDYW GQGTTVTVSSIEVMYPPPYLDNEKSNGTIIHVKGKHLC PSPLFPGPSKPFWVLVVVGGVLACYSLLVTVAFIIFWVR SKRSRLLHSDYMNMTPRRPGPTRKHYQPYAPPRDFAA YRSRVKFSRSADAPAYQQGQNQLYNELNLGRREEYDV LDKRRGRDPEMGGKPRRKNPQEGLYNELQKDKMAEA YSEIGMKGERRRGKGHDGLYQGLSTATKDTYDALHM QALPPR | |
| FLAG-tag | DYKDDDDK | 34 |

## Claims

1. An isolated antibody specifically binding to Glypican-3 (GPC3) protein or a fragment having immunological activity thereof.

2. The isolated antibody or the fragment having immunological activity thereof of claim 1, wherein the antibody comprises a VH domain comprising complementarity determining regions heavy chain 1 (CDRH1) including an amino acid sequence represented by SEQ ID NO: 1, CDRH2 including an amino acid sequence represented by SEQ ID NO: 2, and CDRH3 including an amino acid sequence represented by SEQ ID NO: 3.

3. The isolated antibody or the fragment having immunological activity thereof of claim 1, wherein the antibody comprises a VL domain comprising CDRL1 including an amino acid sequence represented by SEQ ID NO: 4, CDRL2 including an amino acid sequence represented by SEQ ID NO: 5, and CDRL3 including an amino acid sequence represented by SEQ ID NO: 6.

4. The isolated antibody or the fragment having immunological activity thereof of claim 1, wherein the antibody comprises a VH domain including an amino acid sequence represented by SEQ ID NO: 7.

5. The isolated antibody or the fragment having immunological activity thereof of claim 1, wherein the antibody comprises a VL domain including an amino acid sequence represented by SEQ ID NO: 8.

6. The isolated antibody or the fragment having immunological activity thereof of claim 1, wherein the antibody is a chimeric antibody, a bivalent, a bispecific molecule, a minibody, a domain antibody, a bispecific antibody, an antibody mimic, a diabody, a triabody, or a tetrabody.

7. The isolated antibody or the fragment having immunological activity thereof of claim 1, wherein the fragment having immunological activity is any one selected from the group consisting of Fab, Fd, Fab', dAb, F(ab'), F(ab')2, single chain fragment variable (scFv), Fv, a single-chain antibody, an Fv dimer, a complementarity determining region fragment, a humanized antibody, a chimeric antibody, and a diabody.

8. A humanized antibody specifically binding to GPC3 or an antigen binding fragment thereof, comprising:
a human VH domain including any one or more CDRHs selected from the group consisting of complementarity determining regions heavy chain 1 (CDRH1) including an amino acid sequence represented by SEQ ID NO: 1, CDRH2 including an amino acid sequence represented by SEQ ID NO: 2, and CDRH3 including an amino acid sequence represented by SEQ ID NO: 3; and
a human VL domain including any one or more CDRLs selected from the group consisting of CDRL1 including an amino acid sequence represented by SEQ ID NO: 4, CDRL2 including an amino acid sequence represented by SEQ ID NO: 5, and CDRL3 including an amino acid sequence represented by SEQ ID NO: 6.

9. The humanized antibody or the antigen binding fragment thereof of claim 8, wherein the human VH domain is any one or more selected from the group consisting of VH1 including an amino acid sequence represented by SEQ ID NO: 11; VH2 including an amino acid sequence represented by SEQ ID NO: 12; VH3 including an amino acid sequence represented by SEQ ID NO: 13; and VH4 including an amino acid sequence represented by SEQ ID NO: 14.

10. The humanized antibody or the antigen binding fragment thereof of claim 8, wherein the human VL domain is any one or more selected from the group consisting of VL1 including an amino acid sequence represented by SEQ ID NO: 15; VL2 including an amino acid sequence represented by SEQ ID NO: 16; VL3 including an amino acid sequence represented by SEQ ID NO: 17; and VL4 including an amino acid sequence represented by SEQ ID NO: 18.

11. The humanized antibody or the antigen binding fragment thereof of claim 8, wherein the humanized antibody comprises VH1 including an amino acid sequence represented by SEQ ID NO: 11 and VL1 including an amino acid sequence represented by SEQ ID NO: 15; VH1 including an amino acid sequence represented by SEQ ID NO: 11 and VL2 including an amino acid sequence represented by SEQ ID NO: 16; VH1 including an amino acid sequence represented by SEQ ID NO: 11 and VL3 including an amino acid sequence represented by SEQ ID NO: 17; VH1 including an amino acid sequence represented by SEQ ID NO: 11 and VL4 including an amino acid sequence represented by SEQ ID NO: 18; VH2 including an amino acid sequence represented by SEQ ID NO: 12 and VL1 including an amino acid sequence represented by SEQ ID NO: 15; VH2 including an amino acid sequence represented by SEQ ID NO: 12 and VL2 including an amino acid sequence represented by SEQ ID NO: 16; VH2 including an amino acid sequence represented by SEQ ID NO: 12 and VL3 including an amino acid sequence represented by SEQ ID NO: 17; VH2 including an amino acid sequence represented by SEQ ID NO: 12 and VL4 including an amino acid sequence represented by SEQ ID NO: 18; VH4 including an amino acid sequence represented by SEQ ID NO: 14 and VL3 including an amino acid sequence represented by SEQ ID NO: 17; or VH4 including an amino acid sequence represented by SEQ ID NO: 14 and VL4 including an amino acid sequence represented by SEQ ID NO: 18.

12. A hybridoma cell line producing the isolated antibody or the fragment having immunological activity thereof of claim 1 or the humanized antibody or the antigen binding fragment thereof of claim 8.

13. An isolated nucleic acid molecule encoding the isolated antibody or the fragment having immunological activity thereof of claim 1 or the humanized antibody or the antigen binding fragment thereof of claim 8.

14. The isolated nucleic acid molecule of claim 13, wherein the nucleic acid molecule encoding the isolated antibody or the fragment having immunological activity thereof of claim 1 comprises a nucleotide sequence represented by at least any one selected from the group consisting of SEQ ID NOs: 9 and 10.

15. A vector comprising an isolated nucleic acid molecule encoding the isolated antibody or the fragment having immunological activity thereof of claim 1 or the humanized antibody or the antigen binding fragment thereof of claim 8.

16. A host cell transformed with a vector comprising an isolated nucleic acid molecule encoding the isolated antibody or the fragment having immunological activity thereof of claim 1 or the humanized antibody or the antigen binding fragment thereof of claim 8.

17. A composition for detecting cancer antigens comprising the isolated antibody or the fragment having immunological activity thereof of claim 1 or the humanized antibody or the antigen binding fragment thereof of claim 8.

18. The composition for detecting cancer antigens of claim 17, wherein the cancer antigen is GPC3.

19. A kit for detecting cancer antigens comprising the isolated antibody or the fragment having immunological activity thereof of claim 1 or the humanized antibody or the antigen binding fragment thereof of claim 8.

20. An antibody-drug conjugate (ADC) comprising:
the isolated antibody or the fragment having immunological activity thereof of claim 1 or the humanized antibody or the antigen binding fragment thereof of claim 8; and
a drug.

21. The antibody-drug conjugate of claim 20, wherein the drug is an immunogenic apoptosis inducer, a microtubulin structure formation inhibitor, a meiosis inhibitor, a topoisomerase inhibitor, a DNA intercalator, a toxin, or a radionuclide.

22. A pharmaceutical composition for preventing or treating cancer comprising the antibody-drug conjugate of claim 20.

23. The pharmaceutical composition for preventing or treating cancer of claim 22, wherein the cancer is at least any one selected from the group consisting of brain tumor, melanoma, myeloma, non-small cell lung cancer, oral cancer, liver cancer, gastric cancer, colon cancer, breast cancer, triple negative breast cancer (TNBC), lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph node cancer, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma.

24. A composition for diagnosing cancer comprising: the isolated antibody or the fragment having immunological activity thereof of claim 1, the humanized antibody or the antigen binding fragment thereof of claim 8, or the antibody-drug conjugate of claim 20.

25. A kit for diagnosing cancer comprising: the isolated antibody or the fragment having immunological activity thereof of claim 1, the humanized antibody or the antigen binding fragment thereof of claim 8, or the antibody-drug conjugate of claim 20.

26. A method for providing information for the diagnosis of cancer comprising:
treating a sample with the isolated antibody or the fragment having immunological activity thereof of claim 1, the humanized antibody or the antigen binding fragment thereof of claim 8, or the antibody-drug conjugate of claim 20, which is labeled with a fluorescent substance; and
measuring the expression of the isolated antibody or the fragment having immunological activity thereof, the humanized antibody or the antigen binding fragment thereof, or the antibody-drug conjugate.

27. A GPC3-specific chimeric antigen receptor (CAR) comprising:
as an antigen binding domain, a single chain variable fragment (scFv) including a heavy chain variable region including complementarity determining regions heavy chain 1 (CDRH1) including an amino acid sequence represented by SEQ ID NO: 1, CDRH2 including an amino acid sequence represented by SEQ ID NO: 2, and CDRH3 including an amino acid sequence represented by SEQ ID NO: 3; and
a light chain variable region including CDRL1 including an amino acid sequence represented by SEQ ID NO: 4, CDRL2 including an amino acid sequence represented by SEQ ID NO: 5, and CDRL3 including an amino acid sequence represented by SEQ ID NO: 6.

28. The GPC3-specific CAR of claim 27, wherein the heavy chain variable region includes an amino acid sequence represented by SEQ ID NO: 20.

29. The GPC3-specific CAR of claim 27, wherein the light chain variable region includes an amino acid sequence represented by SEQ ID NO: 19.

30. The GPC3-specific CAR of claim 27, wherein the scFv includes an amino acid sequence represented by SEQ ID NO: 22.

31. A GPC3-specific humanized antibody-derived chimeric antigen receptor (CAR) comprising:
as an antigen binding domain, a single chain variable fragment (scFv) including any one heavy chain variable region selected from the group consisting of a heavy chain variable region including an amino acid sequence represented by SEQ ID NO: 11; a heavy chain variable region including an amino acid sequence represented by SEQ ID NO: 12; a heavy chain variable region including an amino acid sequence represented by SEQ ID NO: 13; and a heavy chain variable region including an amino acid sequence represented by SEQ ID NO: 14; and
any one light chain variable region selected from the group consisting of a light chain variable region including an amino acid sequence represented by SEQ ID NO: 15; a light chain variable region including an amino acid sequence represented by SEQ ID NO: 16; a light chain variable region including an amino acid sequence represented by SEQ ID NO: 17; and a light chain variable region including an amino acid sequence represented by SEQ ID NO: 18.

32. The GPC3-specific humanized antibody-derived CAR of claim 31, wherein the scFv comprises any one selected from the group consisting of a heavy chain variable region including an amino acid sequence represented by SEQ ID NO: 11 and a light chain variable region including an amino acid sequence represented by SEQ ID NO: 15; a heavy chain variable region including an amino acid sequence represented by SEQ ID NO: 11 and a light chain variable region including an amino acid sequence represented by SEQ ID NO: 17; a heavy chain variable region including an amino acid sequence represented by SEQ ID NO: 12 and a light chain variable region including an amino acid sequence represented by SEQ ID NO: 16; and a heavy chain variable region including an amino acid sequence represented by SEQ ID NO: 12 and a light chain variable region including an amino acid sequence represented by SEQ ID NO: 18.

33. The GPC3-specific humanized antibody-derived CAR of claim 31, wherein the scFv includes an amino acid sequence represented by any one selected from the group consisting of SEQ ID NOs: 23, 24, 25, and 26.

34. A polynucleotide comprising a nucleic acid sequence encoding the GPC3-specific CAR of claim 27 or the GPC3-specific humanized antibody-derived CAR of claim 31.

35. A vector comprising a polynucleotide comprising a nucleic acid sequence encoding the GPC3-specific CAR of claim 27 or the GPC3-specific humanized antibody-derived CAR of claim 31.

36. A method for producing a GPC3-specific CAR or a GPC3-specific humanized antibody-derived CAR, the method comprising infecting a host cell with a vector including a polynucleotide including a nucleic acid sequence encoding the GPC3-specific CAR of claim 27 or the GPC3-specific humanized antibody-derived CAR of claim 31.

37. A chimeric antigen receptor expressing cell transformed with the GPC3-specific CAR of claim 27.

38. A chimeric antigen receptor expressing cell transformed with the GPC3-specific humanized antibody-derived CAR of claim 31.

39. The chimeric antigen receptor expressing cell of claim 37 or 38, wherein the chimeric antigen receptor expressing cell is a chimeric antigen receptor expressing macrophage (CAR-macrophage), a chimeric antigen receptor expressing T (CAR-T) cell, a chimeric antigen receptor expressing-gamma-delta T (CAR-Gamma-delta T) cell, or a chimeric antigen receptor natural killer (CAR-NK) cell.

40. A pharmaceutical composition for preventing or treating cancer, the pharmaceutical composition comprising the GPC3-specific CAR of claim 27, the GPC3-specific humanized antibody-derived CAR of claim 31, the chimeric antigen receptor expressing cell transformed with the GPC3-specific CAR of claim 37, or the chimeric antigen receptor expressing cell transformed with the GPC3-specific humanized antibody-derived CAR of claim 38.

41. The pharmaceutical composition for preventing or treating cancer of claim 40, wherein the cancer is refractory to immune checkpoint inhibitors.

42. The pharmaceutical composition for preventing or treating cancer of claim 40, wherein the cancer is at least any one selected from the group consisting of brain tumor, melanoma, myeloma, non-small cell lung cancer, oral cancer, liver cancer, gastric cancer, colon cancer, breast cancer, triple negative breast cancer (TNBC), lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph node cancer, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma.

43. A cell therapeutic agent for preventing or treating cancer, the cell therapeutic agent comprising the chimeric antigen receptor expressing cell transformed with the GPC3-specific CAR of claim 37 or the chimeric antigen receptor expressing cell transformed with the GPC3-specific humanized antibody-derived CAR of claim 38.

44. An anticancer adjuvant comprising: the GPC3-specific CAR of claim 27, the GPC3-specific humanized antibody-derived CAR of claim 31, the chimeric antigen receptor expressing cell transformed with the GPC3-specific CAR of claim 37, or the chimeric antigen receptor expressing cell transformed with the GPC3-specific humanized antibody-derived CAR of claim 38.

45. The anticancer adjuvant of claim 44, wherein the anticancer adjuvant comprises reducing refractoriness to cancer immunotherapy.

46. The anticancer adjuvant of claim 45, wherein the cancer immunotherapy is immune checkpoint inhibitors, an immunosuppressant control drug, a cancer vaccine, an immunoadjuvant, an immune cell for cancer treatment, an immune cell activation cofactor, an antibody for cancer treatment, or cytokines required to maintain activity of the immune cell for cancer treatment.

47. The anticancer adjuvant of claim 46, wherein the immune checkpoint inhibitor is an inhibitor of CTLA-4, PD-1, PD-L1, PD-L2, LAG-3, BTLA, B7H3, B7H4, TIM3, KIR, TIGIT, CD47, VISTA or A2aR.

48. A composition for diagnosing cancer comprising: the GPC3-specific CAR of claim 27, the GPC3-specific humanized antibody-derived CAR of claim 31, the chimeric antigen receptor expressing cell transformed with the GPC3-specific CAR of claim 37, or the chimeric antigen receptor expressing cell transformed with the GPC3-specific humanized antibody-derived CAR of claim 38.

49. A method for providing information for the diagnosis of cancer comprising: treating a sample with the GPC3-specific CAR of claim 27, the GPC3-specific humanized antibody-derived CAR of claim 31, the chimeric antigen receptor expressing cell transformed with the GPC3-specific CAR of claim 37, or the chimeric antigen receptor expressing cell transformed with the GPC3-specific humanized antibody-derived CAR of claim 38, which is labeled with a fluorescent substance; and measuring the expression of the GPC3-specific CAR, the GPC3-specific humanized antibody-derived CAR, the chimeric antigen receptor expressing cell transformed with the GPC3-specific CAR, or the chimeric antigen receptor expressing cell transformed with the GPC3-specific humanized antibody-derived CAR.

50. A method for detecting cancer antigens comprising: administering, to a subject, the isolated antibody or the fragment having immunological activity thereof of claim 1, the humanized antibody or the antigen binding fragment thereof of claim 8, the GPC3-specific CAR of claim 27, the GPC3-specific humanized antibody-derived CAR of claim 31, the chimeric antigen receptor expressing cell transformed with the GPC3-specific CAR of claim 37, or the chimeric antigen receptor expressing cell transformed with the GPC3-specific humanized antibody-derived CAR of claim 38.

51. The method for detecting cancer antigens of claim 50, wherein the cancer antigen is GPC3.

52. A method for preventing or treating cancer comprising: the method administering, to a subject, the antibody-drug conjugate of claim 20, the GPC3-specific CAR of claim 27, the GPC3-specific humanized antibody-derived CAR of claim 31, the chimeric antigen receptor expressing cell transformed with the GPC3-specific CAR of claim 37, the chimeric antigen receptor expressing cell transformed with the GPC3-specific humanized antibody-derived CAR of claim 38, a cell therapeutic agent including the chimeric antigen receptor expressing cell transformed with the GPC3-specific CAR of claim 37, or a cell therapeutic agent including the chimeric antigen receptor expressing cell transformed with the GPC3-specific humanized antibody-derived CAR of claim 38.

53. The method for preventing or treating cancer of claim 52, wherein the cancer is refractory to immune checkpoint inhibitors.

54. The method for preventing or treating cancer of claim 52, wherein the cancer is at least any one selected from the group consisting of brain tumor, melanoma, myeloma, non-small cell lung cancer, oral cancer, liver cancer, gastric cancer, colon cancer, breast cancer, triple negative breast cancer (TNBC), lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph node cancer, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma.

55. A method for enhancing an anticancer treatment effect in a subject, the method comprising: administering, to a subject, the GPC3-specific CAR of claim 27, the GPC3-specific humanized antibody-derived CAR of claim 31, the chimeric antigen receptor expressing cell transformed with the GPC3-specific CAR of claim 37, or the chimeric antigen receptor expressing cell transformed with the GPC3-specific humanized antibody-derived CAR of claim 38.

56. The method for enhancing the anticancer treatment effect in the subject of claim 55, wherein the enhancing of the anticancer treatment effect in the subject comprises reducing refractoriness to cancer immunotherapy.
